(19) Europäisches Patentamt
European Patent Office
Office européen des brevets

(11) EP 3 446 720 A1

(12) **EUROPEAN PATENT APPLICATION**
published in accordance with Art. 153(4) EPC

(43) Date of publication:
**27.02.2019 Bulletin 2019/09**

(21) Application number: **17834460.2**

(22) Date of filing: **27.07.2017**

(51) Int Cl.:
***A61L 27/14*** *(2006.01)* ***A61L 27/44*** *(2006.01)*
***A61L 27/50*** *(2006.01)* ***G02C 7/04*** *(2006.01)*

(86) International application number:
**PCT/JP2017/027184**

(87) International publication number:
**WO 2018/021458 (01.02.2018 Gazette 2018/05)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(30) Priority: **28.07.2016 JP 2016148404**

(71) Applicant: **Toray Industries, Inc.**
**Tokyo 103-8666 (JP)**

(72) Inventors:
- **KATO, Tomohiro**
**Otsu-shi**
**Shiga 520-8558 (JP)**
- **NAKAMURA, Masataka**
**Otsu-shi**
**Shiga 520-8558 (JP)**

(74) Representative: **Prüfer & Partner mbB**
**Patentanwälte · Rechtsanwälte**
**Sohnckestraße 12**
**81479 München (DE)**

# (54) MEDICAL DEVICE AND METHOD OF MANUFACTURING MEDICAL DEVICE

(57) A purpose of the present invention is to provide a medical device that not only exhibits superior water-leakage properties and anti-contaminant properties, but also has highly durable lubricity. Another purpose of the present invention is to provide a method of manufacturing said medical device via a simple process without inducing substrate deformation. In order to achieve the aforementioned purposes, the present invention has the following configuration. Specifically, provided is a medical device having a layer containing two or more types of ionic polymer on at least part of the surface of a medical device substrate, at least one of the two or more types of ionic polymer being a block polymer that has a hydrophobic segment.

EP 3 446 720 A1

**Description**

TECHNICAL FIELD

The present invention relates to a medical device having a hydrophilic surface and a method of manufacturing a medical device. Particularly, the present invention relates to a medical device to be suitably used for contact lenses and intraocular lenses and a method of manufacturing a medical device.

BACKGROUND ART

Medical devices that come into direct contact with a part of the human body are well known and their surfaces need to be biocompatible. For development of biocompatibility, it is important that adhesion of substances such as water, protein and lipid is controlled, and chemical modification of the surface is known to be useful. Especially in medical devices that come into contact with living bodies and living body-derived molecules for a long time, durability of biocompatibility is important, and biocompatibility needs to be maintained for a sufficient period of time.

One example of medical devices is a soft contact lens. In recent years, a soft contact lens having high oxygen permeability, containing a compound containing silicon or fluorine is used. The low water content and non-hydrous soft contact lenses are superior in that they have high oxygen permeability, but in order to improve the hydrophobicity of a lens surface, it is necessary to subject the surface to a hydrophilization treatment, and it is important to impart biocompatibilities, namely water wettability, antifouling property and lubricity on the surface. If such biocompatibilities are insufficient, the contact lens adheres to the cornea, not only wearing feeling deteriorates but also the risk of eye disease may be increased by injuring the cornea. It is also preferable that the surface characteristics of the contact lens be maintained over a long period of time, and it is necessary that the contact lens can exhibit sufficient biocompatibility not only during the storage period but also during routine use, and it is important that biocompatibility is not impaired even if routine care, such as scrubbing, is taken.

As described above, it is desirable to provide a contact lens having durability as much as it can withstand routine use and having a surface capable of showing sufficient biocompatibility. A lens having such a surface exhibits good wearing feeling in actual practice and makes it possible to wear a lens for a long time without exerting stimulation to the cornea or harmful influences. It is more desirable that economical and commercial production of such a lens can be realized relatively easily.

Materials having such superior characteristics are suitable for use not only for ophthalmic lenses but also for medical devices such as artificial kidneys, endoscopes, catheters, infusion tubes, gas delivery tubes, stents, sheaths, cuffs, tube connectors, access ports, drainage bags, blood circuits, skin materials or drug carriers.

Various methods are known for surface treatment of medical devices, particularly contact lenses, and some examples are given below. Patent Documents 1 and 2 disclose a method of applying layered coating to a surface of a medical device by immersing the device in an aqueous solution of a hydrophilic polymer. Patent Documents 3 and 4 disclose a method of adding a hydrophilic polymer having a silicone segment as a wetting agent to a packaging solution. Patent Document 5 discloses a method of fixing a hydrophilic coating polymer on a surface by chemical covalent bonding.

PRIOR ART DOCUMENTS

PATENT DOCUMENTS

Patent Document 1: WO 2013/024801
Patent Document 2: JP-A-2005-538767
Patent Document 3: JP-A-2013-532196
Patent Document 4: JP-A-2012-246489
Patent Document 5: JP-A-2007-206166

SUMMARY OF THE INVENTION

PROBLEMS TO BE SOLVED BY THE INVENTION

As described above, it is important for a medical device surface to have sufficiently durable biocompatibilities (water wettability, antifouling property, and lubricity). For example, plasma surface treatment is known as a method for imparting biocompatibilities to a surface of a medical device, but the effect is generally only temporary, and it has been

difficult to maintain surface characteristics. In addition, plasma surface treatment requires a large-scale apparatus and a special process, which is complicated and industrially disadvantageous.

On the other hand, in the methods disclosed as examples in Patent Documents 1 and 2, it is possible to obtain a hydrophilic surface composed of a plurality of layers by combining acidic/basic polymers or polymers having a charge/not having any charge. Generally, it is considered that such layered coating is relatively strongly fixed by electrostatic interaction and has high durability. However, in the case where the medical device substrate has high hydrophobicity and low affinity with the hydrophilic polymer, there are cases in which formation of the coating layer is incomplete and coarse/dense coating occurs or durability is insufficient.

According to the methods disclosed as examples in Patent Documents 3 and 4, it is possible to introduce a block having affinity with a substrate into a hydrophilic polymer and impart wettability to a hydrophobic surface of a substrate. However, in this method, since a polymer is merely adhered to a surface of a substrate due to a relatively weak interaction, the effect does not last long and the durability is insufficient in some cases. In addition, since the block having affinity with a substrate is hydrophobic, water wettability may be lowered when a hydrophobic part appears on the surface.

In the method disclosed as an example in Patent Document 5, although a coating layer is firmly held on a surface by covalent bonding, it requires a step of performing a chemical reaction for forming a covalent bonding, which is not only complicated, but also deformation of a substrate may be induced due to use of a solvent.

In view of the above problems, it is an object of the present invention to provide a medical device having not only superior water wettability and antifouling property but also having high durability of lubricity. It is another object of the present invention to provide a method of manufacturing such a medical device by a simple process without inducing deformation of a substrate.

SOLUTIONS TO THE PROBLEMS

In order to achieve the above objects, the inventors of the present invention conducted intensive studies and found that by using in combination an ionic polymer having a block having affinity with a substrate and one or more ionic polymers, it is possible to improve the durability of a hydrophilic layer more strongly containing such an ionic polymer while suppressing formation of a surface with poor water wettability. That is, the present invention has the following configurations.

[1] A medical device having a layer containing two or more ionic polymers on at least a part of a surface of a medical device substrate, wherein at least one of the two or more ionic polymers is a block polymer having a hydrophobic segment.

[2] The medical device according to [1], wherein the layer containing two or more ionic polymers contains one or more ionic polymers having a positive charge and one or more ionic polymers having a negative charge.

[3] The medical device according to [1], wherein the layer containing two or more ionic polymers includes one or more layers containing one or more ionic block polymers having the hydrophobic segment and one or more layers containing one or more ionic polymers having no hydrophobic segment.

[4] The medical device according to [3], wherein the one or more layers containing one or more ionic polymers having no hydrophobic segment contains an ionic polymer having a charge opposite to that of the ionic block polymer having a hydrophobic segment

[5] The medical device according to any one of [1] to [4], wherein a mass-average molecular weight of the block polymer having a hydrophobic segment is within a range of 10,000 to 10,000,000.

[6] The medical device according to any one of [1] to [5], wherein the hydrophobic segment contains a polydimethylsiloxane structure.

[7] The medical device according to any one of [1] to [6], wherein a structure of the block polymer having the hydrophobic segment is represented by the formula (b1):

[Chemical formula 1]

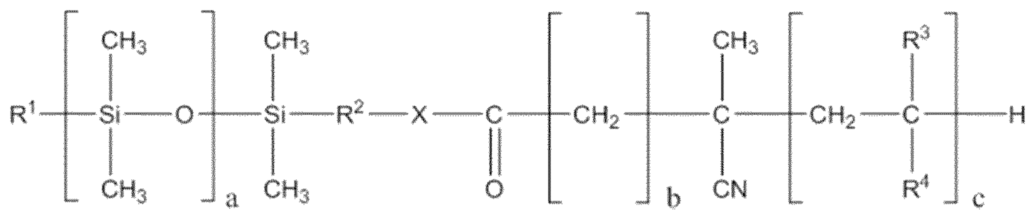

(b1)

in the formula (b1), $R^1$ is an alkyl group or an alkoxy group, $R^2$ is $(CH_2)_n$ or $(CH_2)_m$-$O(CH_2)_n$, m and n are each independently an integer of 1 to 16, a is 4 to 19, b is 1 to 6, c is 1 to 10000, X is O, NH, or S, $R^3$ represents H or $CH_3$, $R^4$ contains one or more structures represented by the formulae (a1) to (a3):

[Chemical formula 2]

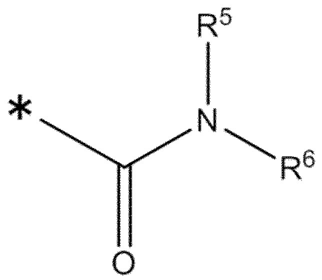
(a 1)

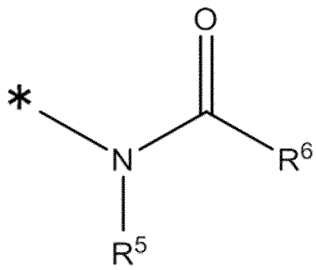
(a 2)

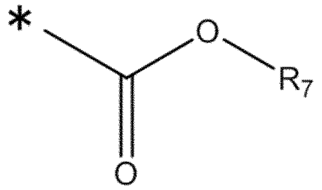
(a 3)

in the formulae (a1) to (a3), each * represents a bonding site to a polymer backbone; in the formulae (a1) to (a3), $R^5$ to $R^7$ are each independently a hydrogen atom or an alkyl group having 1 to 20 carbon atoms that may be branched or linear and optionally has a cyclic structure and optionally is substituted; $R^5$ and $R^6$ may form a ring via a bond.
[8] The medical device according to any one of [1] to [7], wherein the block polymer having the hydrophobic segment contains 0.01 to 10% by mass of the hydrophobic segment and further contains 90 to 99.9% by mass of a hydrophilic segment.
[9] The medical device according to any one of [1] to [8], wherein repeating units other than hydrophobic segments in the block polymer having the hydrophobic segment include at least one amide structure.
[10] The medical device according to any one of [1] to [9], wherein the block polymer having the hydrophobic segment contains at least one structure selected from structures derived from N-vinylpyrrolidone or structures derived from N,N-dimethylacrylamide.
[11] The medical device according to any one of [1] to [10], which is an ophthalmic lens.
[12] The medical device according to [11], wherein the ophthalmic lens is a contact lens.
[13] A method of manufacturing a medical device according to any one of [1] to [12], the method including the following steps 2a to 3a in this order:

<Step 2a>
the step of bringing at least a part of a medical device substrate into contact with a solution of an ionic block polymer having a hydrophobic segment and then removing a surplus polymer solution;
<Step 3a>
the step of bringing at least a part of the medical device substrate into contact with a solution of an ionic polymer and then removing a surplus polymer solution.

[14] A method of manufacturing a medical device according to any one of [1] to [12], the method including the following steps 1a to 3a in this order:

<Step 1a>
the step of synthesizing an ionic block polymer having a hydrophobic segment;
<Step 2a>
the step of bringing at least a part of a medical device substrate into contact with a solution of an ionic block polymer having a hydrophobic segment and then removing a surplus polymer solution;

<Step 3a>
the step of bringing at least a part of the medical device substrate into contact with a solution of an ionic polymer and then removing a surplus polymer solution.

[15] The method according to [13] or [14], wherein the ionic polymer in the Step 3a has a charge opposite to the ionic block polymer having a hydrophobic segment.

EFFECTS OF THE INVENTION

The medical device of the present invention is one having not only superior water wettability and antifouling property but also having high durability of lubricity. In addition, it is possible to manufacture such a medical device by the method of manufacturing a medical device of the present invention without inducing deformation of a substrate.

BRIEF DESCRIPTION OF THE DRAWINGS

Fig. 1 is an AFM observed image (1) of a surface of a coated lens.
Fig. 2 is an AFM observed image (2) of a surface of a coated lens.

EMBODIMENTS OF THE INVENTION

The medical device of the present invention can enhance affinity with a substrate and can provide a biocompatible surface with improved durability by using an ionic block polymer having a hydrophobic segment and one or more ionic polymers in combination.
The medical device referred to in the present invention means a device that is used for medical use and is used by being in contact with a patient or being in contact with a tissue taken from a patient such as blood or other body fluids. Preferable examples thereof include ophthalmic lenses, endoscopes, catheters, infusion tubes, gas transport tubes, stents, sheaths, cuffs, tube connectors, access ports, drainage bags, blood circuits, skin materials, and drug carriers. It is particularly suitable for ophthalmic lenses. Examples of ophthalmic lenses include contact lenses such as soft contact lenses, hard contact lenses and hybrid contact lenses, scleral lenses, intraocular lenses, artificial cornea, corneal inlays, corneal onlays, and eyeglass lenses. Among these, it is suitable for contact lenses and is particularly suitable for soft contact lenses. As a soft contact lens, it is suitable for a high water content lens having a water content of 40% or more and a low water content lens having a water content of less than 40% and a non-hydrous lens, but it can be particularly preferably used for a low water content lens and a non-hydrous lens.
The medical device substrate in the present invention refers to a material constituting the medical device and may be either a part or the whole of the medical device excluding the layer containing two or more ionic polymers.
The ionic polymer in the present invention refers to an ionic polymer in which at least one functional group present in the polymer can participate in donation and reception of protons in a protic solvent such as water. More specifically, it refers to a polymer that has a positive or negative potential, in other words, has a positive or negative charge when a zeta-potential thereof is measured in pure water. The ionic polymer in the present invention is preferably a hydrophilic polymer from the viewpoint that good biocompatibilities can be obtained.
In the present invention, the hydrophilic polymer corresponds to one or both of the following (D-1) and (D-2) :

(D-1) a polymer that dissolves to 1 g or more in 100 g of water at 20°C
(D-2) a polymer in which structural units derived from hydrophilic monomers account for 10 mol% or more of the structural units derived from the plurality of monomers constituting the polymer.

As (D-1), a polymer that dissolves to 10 g or more is more preferred, a polymer that dissolves to 50 g or more is even more preferred, a polymer that dissolves to 100 g or more is still even more preferred, and a hydrophilic monomer that is miscible with water in any proportions without separating from water into layers is particularly preferred. A hydrophilic polymer which is dissolved in water to form an acidic or basic aqueous solution is more preferred.
As (D-2), a polymer containing structural units derived from hydrophilic monomers in 15 mol% or more of the structural units derived from the plurality of monomers constituting the polymer is more preferred, and a polymer containing structural units derived from hydrophilic monomers in 20 mol% or more is even more preferred. From the viewpoint that good water wettability can be obtained, a polymer containing structural units derived from hydrophilic monomers in 30 mol% or more is still even more preferred, and a polymer containing structural units derived from hydrophilic monomers in 50 mol% or more is more preferred.

The term "layer" referred to in the present invention means an aggregate of molecules formed on a substrate surface. In the present invention, layers are not required to have a structure that is microscopically uniform in its horizontal direction or depth direction and also are not required to have a structure in which polymers are stacked flat. For example, there may be micro portions where no polymers are present, in the present invention. Moreover, the polymer and the substrate may be present in admixture or a clear interface between layers may not be present.

In the present invention, the layer containing two or more ionic polymers may also be referred to as a coating layer.

In the present invention, the term "hydrophobic segment" means a part of a molecular chain containing a structure derived from a repeating unit having a hydrophobic residue, and hydrophobic segments are those which do not yield a clear single phase when mixed with water at 2000 ppm at 25°C. When carrying out this measurement, each end of the hydrophobic segment may be independently substituted with a hydrogen atom or an initiator residue or a functional group. Examples of suitable hydrophobic segments are polysiloxanes, C8-C50 alkylene or (poly)arylene groups, hydrophobic polymers formed from monomers selected from the group consisting of C1-C20 alkyl or C6-C20 aryl(meth)acrylate monomers such as methyl(meth)acrylate, ethyl(meth)acrylate, n-propyl (meth)acrylate, isopropyl(meth)acrylate, n-butyl (meth)acrylate, n-decyl(meth)acrylate, n-dodecyl (meth)acrylate, phenyl(meth)acrylate, and naphthyl (meth)acrylate; and silicone(meth)acrylate monomers such as 3-(meth)acryloxypropyltris(trimethylsiloxy)silane, pentamethyldisiloxanylmethyl(meth)acrylate, methyldi(trimethylsiloxy)(meth)acryloxymethylsilane, mono(meth)acryloxypropyl terminated mono-n-butyl terminated polydimethylsiloxane, (2-methyl-)2-propenoic acid, 2-hydroxy-3-[3-[1,3,3,3-tetramethyl-1-[trimethylsilyl)oxy]disiloxanyl]propoxy]propyl ester, and 9-n-butyl-1-[3-(3-(meth)acryloyloxy-2-hydroxypropoxy)propyl]-1,1,3,3,5,5,7,7,9,9-decamethylpentasiloxane ; and vinyl or allyl silicone monomers such as 3-[tris(trimethylsiloxy)silyl]propyl allyl carbamate, 3-[tris(trimethylsiloxy)silyl]propyl vinyl carbamate, trimethylsilylethyl vinyl carbonate, trimethylsilylmethyl vinyl carbonate; and aromatic vinyl monomers such as styrene, and vinylpyridine; and combinations thereof. In the present invention, the hydrophobic segment of the block polymer is preferably a polysiloxane segment. The polysiloxane segment may contain C1-C4 polyalkyl and polyaryl substituted siloxane repeating units. Examples of suitable polysiloxane repeating units include polydimethylsiloxane, polydiethylsiloxane, polydiphenylsiloxanes and copolymers thereof, and polydimethylsiloxane is preferable in terms of its easy availability. In other words, in the medical device of the present invention, the hydrophobic segment preferably contains a polydimethylsiloxane structure. The polysiloxane segment is preferably one having an alkyl group, more preferably one having an alkyl group having 1 to 4 carbon atoms, and particularly preferably one having a methyl group or a n-butyl group at one end.

In the present invention, specific examples of the hydrophobic segment preferably include those containing a structure represented by the formula (b2).

[Chemical formula 3]

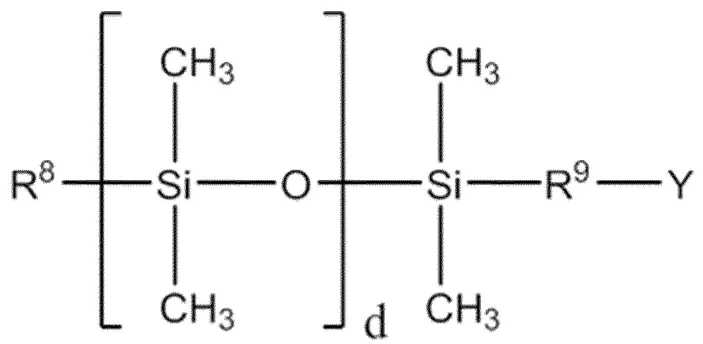

(b2)

In the formula (b2), $R^8$ is an alkyl group or an alkoxy group, $R^9$ is $(CH_2)_n$ or $(CH_2)_m$-$O(CH_2)_n$ where m and n are each independently an integer of 1 to 16, d is 4 to 19, and Y is O, NH, or S.

In the formula (b2), the lower limit of the number of carbon atoms of the alkyl group or the alkoxy group as $R^8$ is preferably 1, more preferably 2, even more preferably 3, and particularly preferably 4. The upper limit is preferably 15, more preferably 12, even more preferably 9, and particularly preferably 6. Any of the upper limits may be combined with any of the lower limits.

In the formula (b2), the lower limits of m and n in $R^9$ are each independently preferably 1, more preferably 2, and particularly preferably 3. The upper limits are each independently preferably 10, more preferably 8, and particularly preferably 6.

In the formula (b2), the lower limit of d is preferably 4, more preferably 5, and particularly preferably 6. The upper limit is preferably 19, more preferably 18, and particularly preferably 17. d may have a distribution, and having a distribution in the present description means a mixture of molecules having a plurality of values of d.

The block polymer having a hydrophobic segment in the present invention is an ionic polymer, and preferably is a hydrophilic polymer as an overall molecular chain. Therefore, the block polymer having a hydrophobic segment in the present invention is preferably a block polymer having both a hydrophobic segment and a hydrophilic segment. The hydrophilic segment as referred to herein means a hydrophilic residue having a structure containing a repeating unit and refers to one that satisfies the definition of the hydrophilic polymer described above. In the present description, a part of a block polymer having a hydrophobic segment other than the hydrophobic segment may be referred to as a hydrophilic segment.

In the present invention, the block polymer having a hydrophobic segment preferably contains 0.01 to 10% by mass of a hydrophobic segment and 90 to 99.9% by mass of a hydrophilic segment and more preferably contains 0.01 to 5% by mass of at least one hydrophobic segment and 95 to 99.99% by mass of a hydrophilic segment, based on the mass-average molecular weight of the block polymer. When the mass ratio of hydrophobic segments is excessively small, the effect of the present invention may be difficult to obtain because the affinity with a substrate is lowered, whereas when the mass ratio is excessively large, the biocompatibility and the solubility in water of the polymer itself may be deteriorated due to deterioration in hydrophilicity. Accordingly, in the present invention, the lower limit of the mass ratio of hydrophobic segments in the block polymer having a hydrophobic segment is preferably 0.001% by mass, more preferably 0.01% by mass, even more preferably 0.02% by mass, particularly preferably 0.05% by mass, and most preferably 0.1% by mass. The upper limit is preferably 10% by mass, more preferably 8% by mass, even more preferably 5% by mass, and particularly preferably 3% by mass. Any of the upper limits may be combined with any of the lower limits. The upper limit of the mass ratio of hydrophilic segments in the corresponding block polymer having a hydrophilic segment is preferably 99.999%, more preferably 99.98%, and even more preferably 99.95%, and particularly preferably 99.9%. The lower limit is preferably 90%, more preferably 92%, even more preferably 95%, and particularly preferably 97%. Any of the upper limits may be combined with any of the lower limits.

In the present invention, a block polymer refers to a polymer including at least two different repeating structural units and may have three or more blocks, but from the viewpoint of ease of synthesis, a block polymer or a triblock polymer is preferred, and a diblock polymer is more preferred. In the present invention, the block polymer may be either linear or branched. Each block may be composed of structural units derived from two or more types of monomers, that is, it may be copolymerized, and in this case, the monomer sequence composition in the block is not necessarily required to be uniform and random, and for example, some gradient may be generated.

In the case where the block polymer having a hydrophobic segment in the present invention has three or more types of blocks, the block polymer may contain a plurality of hydrophobic segments as long as it necessarily contains one hydrophobic segment. The hydrophobic segment in the polymer chain may be located anywhere in the polymer chain, but from the viewpoint that interaction between the hydrophobic segment and the substrate is difficult to be disturbed, the hydrophobic segment exists at an end of the polymer chain.

The block polymer having a hydrophobic segment in the present invention is an ionic polymer, and it is preferable to have structural units derived from at least one ionic monomer in the polymer chain. However, when the block polymer having a hydrophobic segment in the present invention has three or more types of blocks, it is just required to have at least one type of block containing a structural unit derived from an ionic monomer, and it may have hydrophobic or hydrophilic blocks that are substantially nonionic as a third block.

The mass-average molecular weight of the block polymer having a hydrophobic segment is preferably within a range of 10,000 to 10,000,000. If the mass-average molecular weight is excessively small, a coating layer may be difficult to be formed. If the mass-average molecular weight is excessively large, increase in solution viscosity will be induced and the operability may be deteriorated. Therefore, the lower limit of the mass-average molecular weight of the block polymer having a hydrophobic segment is preferably 10,000, more preferably 20,000, even more preferably 50,000, and particularly preferably 100,000. The upper limit is preferably 8,000,000, more preferably 5,000,000, even more preferably 2,000,000, and particularly preferably 1,000,000. Any of the upper limits may be combined with any of the lower limits.

The structure of the block polymer having a hydrophobic segment is preferably represented by the following formula (b1).

[Chemical formula 4]

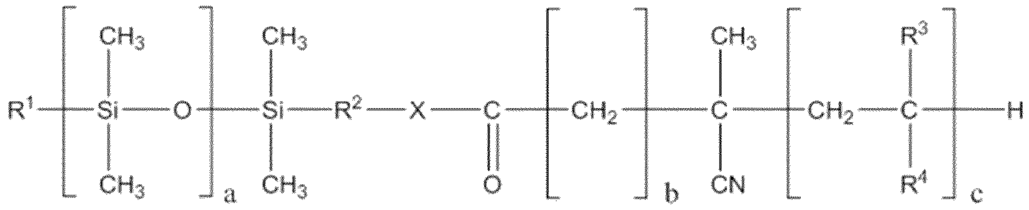

(b1)

In the formula (b1), $R^1$ is an alkyl group or an alkoxy group, $R^2$ is $(CH_2)_n$ or $(CH_2)_m$-$O(CH_2)_n$, m and n are each independently an integer of 1 to 16, a is 4 to 19, b is 1 to 6, c is 1 to 10000, X is O, NH, or S, $R^3$ represents H or $CH_3$, $R^4$ contains one or more structures represented by the formulae (a1) to (a3).

[Chemical Formula 5]

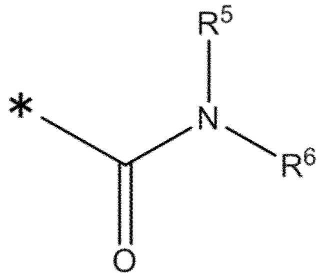

(a 1)

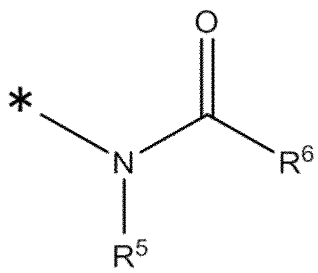

(a 2)

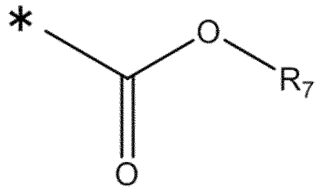

(a 3)

In the formulae (a1) to (a3), each * represents a bonding site to a polymer backbone; in the formulae (a1) to (a3), $R^5$ to $R^7$ are each independently a hydrogen atom or an alkyl group having 1 to 20 carbon atoms that may be branched or linear and optionally has a cyclic structure and optionally is substituted; $R^5$ and $R^6$ may form a ring via a bond.

Preferred embodiments and range of $R^1$, m, n, and a in the formula (b1) are the same as those of $R^8$, m, n and d in the formula (b2).

The part other than the hydrophobic segment of the block polymer having a hydrophobic segment according to the present invention preferably contains a structure derived from a monomer having a polymerizable group.

The polymerizable group referred to in the present invention means a functional group that participates in a polymerization reaction, and from the viewpoint that a protic solvent can be used, it is preferably a radically polymerizable functional group. Examples of preferable polymerizable groups include a vinyl group, an allyl group, a (meth)acryloyloxy group, a (meth)acrylamide group, an $\alpha$-alkoxymethylacryloyloxy group, a maleic acid residue, a fumaric acid residue, an itaconic acid residue, a crotonic acid residue, an isocrotonic acid residue, and a citraconic acid residue. Among these, a (meth)acrylamide group or a (meth)acryloyloxy group is particularly preferred because of their high polymerizability.

The term "(meth)acryloyloxy" as used in the present description represents both methacryloyloxy and acryloyloxy. "(Meth)acrylaminde" can also be interpreted likewise.

In the present invention, preferable types of the monomer to constitute the part other than the hydrophobic segment of the block polymer having a hydrophobic segment, namely, the hydrophilic segment, include (meth)acrylates, (meth)acrylamides, N-vinylcarboxamides, cyclic N-vinyllactams, cyclic N-vinylpyridines, and N-vinylimidazoles, and from the viewpoint that polymerization proceeds at a high rate and a polymer with a uniform composition can be obtained easily, (meth)acrylates and (meth)acrylamides are preferable.

In the present invention, the hydrophilic segment of the block polymer having a hydrophobic segment is required

to be ionic as previously described, and preferably has a positive charge or a negative charge, and such a positive charge or a negative charge is derived from a specific group contained in the hydrophilic segment. The specific group that affords such a positive charge or a negative charge is preferably contained in a structure derived from a radically polymerizable monomer.

As the group that affords a positive charge, a basic functional group can be preferably used, and an amino group and a salt thereof are preferable, and examples of suitable monomers containing such a group include allylamine derivatives, aminostyrene derivatives, amino group-containing (meth)acrylates such as N,N-dialkylaminoethyl methacrylate, amino group-containing (meth)acrylamides such as poly(N,N-dimethylaminopropylacrylamide), N-vinylimidazole derivatives, and salts thereof. Among these, monomers containing a quaternary ammonium salt or an imidazolium salt are particularly preferable because they have antibacterial properties.

As a group that affords a negative charge, acidic functional groups can be preferably used, and a carboxy group, a sulfo group ($-SO_3H$), a sulfate group ($-OSO_3H$)), a phosphonic acid group ($-PO(OH)_2$), a phosphoric acid group ($-OPO(OH)_2$), and salts thereof are preferable. Examples of monomers containing such groups include (meth)acrylic acid, vinylbenzoic acid, styrenesulfonic acid, vinylsulfonic acid, 2-acrylamido-2-methylpropanesulfonic acid, phosphate-based (meth)acrylate monomers, and salts thereof. Of these, (meth)acrylic acid, 2-acrylamido-2-methylpropanesulfonic acid, and salts thereof are more preferable, and (meth)acrylic acid and salts thereof are particularly preferable.

The above-mentioned monomers that afford a positive charge or a negative charge may be used singly to form a homopolymer-like hydrophilic block, but they may form a copolymer through copolymerization with another hydrophilic monomer as a nonionic hydrophilic component. Preferable examples of the monomer of the nonionic hydrophilic component include (meth)acrylate monomers such as 2-hydroxyethyl(meth)acrylate, 2-(2-hydroxyethoxy)ethyl (meth)acrylate, glyceryl(meth)acrylate and poly(ethylene glycol) mono(meth)acrylate, polymerizable carboxylic acid monomers such as (meth)acrylic acid, itaconic acid, crotonic acid, and vinylbenzoic acid, N-vinylamide monomers such as N-vinylpyrrolidone, N-vinylformamide, N-vinylacetamide, N-vinyl-4-methyl-2-caprolactam, and N-vinyl-N-methylacetamide, (meth)acrylamide monomers such as (meth)acrylamide, N,N-dimethyl(meth)acrylamide, N,N-diethyl(meth)acrylamide, N,N-diisopropylacrylamide, N-methylacrylamide, N-ethylacrylamide, N-isopropyl(meth)acrylamide, (meth)acryloyl morpholine, N-methoxymethyl(meth)acrylamide, N-hydroxymethylacrylamide, and diacetone acrylamide. Among these, N-methylacrylamide, N,N-dimethylacrylamide, N-vinylpyrrolidone, N-vinylcaprolactam, N-vinylacetamide, and N-vinyl-N-methylacetamide are more preferable from the viewpoint that hydrophilic polymers superior in water wettability and lubricity can be obtained. N,N-dimethylacrylamide and N-vinylpyrrolidone are particularly preferable from the viewpoint that particularly superior hydrophilicity can be obtained. That is, in the medical device of the present invention, it is preferable that the block polymer having a hydrophobic segment contains at least one structure selected from structures derived from N-vinylpyrrolidone or structures derived from N,N-dimethylacrylamide. The nonionic hydrophilic component monomers may be used singly or two or more of them may be used in combination.

The monomer constituting the hydrophilic segment may be either ionic or nonionic, and from the viewpoint of being particularly superior in water wettability and lubricity, it is preferable to use a monomer having an amide structure. That is, in the medical device of the present invention, it is preferable that at least one amide structure is contained in repeating units other than the hydrophobic segment in the block polymer having a hydrophobic segment.

In the present invention, the "amide structure" refers to a structure represented by the following formula (a). Accordingly, for example, amide compounds, imide compounds, urea compounds, and derivatives thereof have an amide structure.

[Chemical Formula 6]

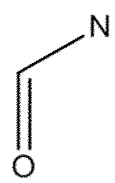

(a)

Among amide structures, an amide bonding is particularly preferable because it has high affinity with water to afford superior water wettability and lubricity and also has superior durability because of its superior hydrolysis resistance as compared with an ester linkage.

The block polymer having a hydrophobic segment according to the present invention may be produced according to any method disclosed in JP-A-2012-246489. A hydrophobic segment-containing macroinitiator may be formed by reacting a reactive linear polysiloxane having a functional group such as a hydroxyl group, amino group, thiol group or

the like on at least one terminus with an azo-type initiator having a carboxy group.

Azo-type initiators are known in the art, and examples thereof include aliphatic azo-containing initiators containing one or more of the following compounds including 4,4'-azobis(4-cyanovaleric acid) and its derivatives, 2,2'-azobis[N-(2-carboxyethyl)-2-methylpropionamidine]hydrate, 2,2'-azobis{2-methyl-N-[2-(1-carboxybutyl)]propionamide}, and 2,2'-azobis[2-methyl-N-(2-carboxyethyl)propionamide]. In one embodiment, 4,4'-azobis(4-cyanovaleric acid) may preferably be used as the azo-type initiator.

It is desirable to control the ratio of the reactive linear polysiloxane to the azo-type initiator to be fed in the reaction. If the reactive linear polysiloxane/the azo-type initiator molar ratio is excessively high, siloxane raw material will remain after reaction, and purifying will be difficult, but if the ratio is excessively low (excessively much initiator), the yield will be reduced. Therefore, the lower limit of the reactive linear siloxane/the azo-type initiator molar ratio is preferably 1.0, more preferably 1.3, and particularly preferably 1.4. The upper limit is preferably 2.4, more preferably 2.0, and particularly preferably 1.9. Any of the upper limits may be combined with any of the lower limits.

The azo-type initiator and the reactive linear polysiloxane are preferably reacted via a condensation reaction at a sufficiently low temperature that the azo-type initiator does not generate radicals.

If the reaction temperature is excessively high, radicals will be generated from the azo-type initiator, but if the temperature is excessively low, a long time will be required until the reaction is complete. Therefore the lower limit of the reaction temperature is preferably -20°C, more preferably 0°C, and particularly preferably 10°C, and the upper limit is preferably 50°C, more preferably 40°C, and particularly preferably 35°C. Any of the upper limits may be combined with any of the lower limits. In the condensation reaction, a condensation agent is preferably used. Examples of the condensation agent include dicyclohexyl carbodiimide (DCC), diisopropyl carbodiimide (DIPC), and N-ethyl-N'-3-dimethyl aminopropyl carbodiimide (EDC=WSCI), as well as hydrochloride salts (WSCI·HCl).

When performing an amide condensation reaction, a combination of DCC or WSCI and N-hydroxysuccinimide (HONSu), 1-hydroxybenzotriazole (HOBt), or 3-hydroxy-4-oxo-3,4-dihydro-1,2,3-benzotriazine (HOOBt) and the like can also be used. If the amount used is excessively low, raw material will remain and purifying will be difficult, but if the amount is excessively high, the condensation agent will remain and purifying will be difficult. Therefore, the lower limit of the molar ratio added is preferably 1.8, more preferably 2.0, and particularly preferably 2.1 the amount of azo-type initiator having a carboxyl group, and the upper limit is preferably 4.0 times, more preferably 3.0 times, and particularly preferably 2.7 times. Any of the upper limits may be combined with any of the lower limits.

When performing an esterification or thioesterification reaction, a catalyst may be added during a macroinitiator synthesis reaction in order to enhance reactivity. Suitable catalysts include 4-dimethylaminopyridine. If the amount used is excessively low, much time will be required for the reaction, but if the amount is excessively high, removing the catalyst after the reaction will be difficult. Therefore, the lower limit of the molar ratio of the catalyst to the initiator is preferably 0.01, more preferably 0.05, and particularly preferably 1.0, and the upper limit is preferably 4.0, more preferably 3.0, and particularly preferably 2.7.

In the present invention, the hydrophobic segment-containing macroinitiator is reacted with at least one hydrophilic monomer to form the block polymer. The hydrophilic segment may be formed from known hydrophilic monomers.

The hydrophilic monomer should be present in a concentration sufficient to achieve a desired degree of polymerization of the hydrophilic segment. If the concentration of the hydrophilic monomer is excessively high, the viscosity becomes high during the polymerization and it will be difficult or, in some cases, impossible to mix. Therefore, the concentration in percent by mass is preferably 10 to 60% by mass, and particularly preferably 15 to 50% by mass.

If the monomer/initiator ratio is excessively low, gelation tends to occur during the polymerization, and if the ratio is excessively high, the polymerization does not start. Therefore, the lower limit of the ratio is preferably 500, more preferably 800, and particularly preferably 1500, and the upper limit is preferably 10,000, more preferably 7,000, and particularly preferably 5,000. Any of the upper limits may be combined with any of the lower limits.

The polymerization may be carried out neat or with a solvent, but it is preferred to use a solvent in order to avoid the risk of reaction runaway, etc. Suitable solvents include ethers, esters, amides, aromatic and aliphatic hydrocarbons, alcohols, ketone solvents, ester solvents, ether solvents, sulfoxide solvents, amide solvents, and glycol solvents and halogenated hydrocarbons. Among these, from the viewpoint of hard to inhibit radical polymerization, more preferable are water and alcohol solvents, and particularly preferable are water and tertiary alcohol solvents. Example include tert-amyl alcohol, diethyl ether, tetrahydrofuran, hexanes, methylene chloride, ethyl acetate, dimethyl formamide, water, methanol, ethanol, propanol, 2-propanol, butanol, tert-butanol, 3-methyl-3-pentanol, 3,7-dimethyl-3-octanol, benzene, toluene, xylene, hexane, heptane, octane, decane, petroleum ether, kerosene, ligroin, paraffin, acetone, methyl ethyl ketone and methyl isobutyl ketone, ethyl acetate, butyl acetate, methyl benzoate, dioctyl phthalate, ethylene glycol diacetate, diethyl ether, tetrahydrofuran, dioxane, dimethyl sulfoxide, N,N-dimethylformamide, N,N-dimethylacetamide, ethylene glycol dialkyl ether, diethylene glycol dialkyl ether, triethylene glycol dialkyl ether, tetraethylene glycol dialkyl ether, polyethylene glycol dialkyl ether, polyethylene glycol-polypropylene glycol block copolymer, and polyethylene glycol-polypropylene glycol random copolymer, and mixtures thereof. Among these, from the viewpoint of hard to inhibit radical polymerization, more preferable are water, tert-butanol, tert-amyl alcohol, 3-methyl-3-pentanol and 3,7-dimethyl-3-octanol. Regarding the addition amount when using a solvent, the lower limit thereof is preferably 40% by mass, more preferably 50% by mass, and particularly preferably 60% by mass, based on the total mass of the hydrophobic segment-containing macroinitiator and the monomers. The upper limit is preferably 90% by mass, more preferably 85% by mass, and particularly preferably 80% by mass. Any of the upper limits may be combined with any of the lower limits.

Any temperature where the selected initiator is active, and between the freezing and boiling point of the reaction components (including solvent, if used) may be used. If the temperature is too high, the polymer solution may also heat excessively and become difficult to control or dangerous. Temperature ranges between the 10 hour half-life temperature of the polymerization initiator (hereinafter referred to as T) and T + 50°C, and in some embodiments between T and T + 30°C are suitable.

Suitable reaction times include up to about 72 hours, but the lower limit of reaction time is preferably 1 hour, more preferably 2 hours, and particularly preferably 3 hours, and the upper limit is preferably 72 hours, more preferably 48 hours, and particularly preferably 24 hours. Any of the upper limits may be combined with any of the lower limits.

The resulting block polymer may be purified via column chromatography, precipitation, washing off impurities by solvent which the block copolymer is insoluble to, fractionation by GPC, or any other traditional means of polymer isolation.

The hydrophobic segment thus obtained has an affinity with at least a part of the medical device, especially a hydrophobic site of the surface of the medical device, and has an action of associating with at least a part of the surface of the medical device. That is, in the present invention, a block polymer having a hydrophobic segment can come into contact with and adhere to the surface of the medical device by making use of the affinity between the hydrophobic segment and the medical device substrate. It is believed that block polymer has enhanced affinity with a substrate because of having a hydrophobic segment, so that defects are less likely to occur in the coating layer and durability can be improved.

Although the affinity with a medical device surface is mainly due to association derived from hydrophobic interaction, it can be attained further by entanglement (entanglement of molecules), van der Waals force, interaction between dipoles, electrostatic attraction (electrostatic interaction), hydrogen bonding, $\pi$-$\pi$ stacking, and combinations of these effects.

Therefore, in the present invention, the coating layer is not required to have a covalent bonding with the substrate. It is preferable that the coating layer does not have a covalent bonding with the substrate because it can be manufactured by a simple process. Even if the coating layer does not have a covalent bonding with the substrate, it will have practical durability by using an ionic polymer described below in combination.

In the present invention, the layer containing two or more ionic polymers may be mixed to form a single layer or may be in a multilayer form composed of a plurality of layers. In particular, from the viewpoint of durability against delamination of the coating layer from the substrate, the form of the layer is preferably a multilayer form. The form of the layer can be analyzed by staining with ruthenium tetraoxide or the like and observing with a transmission electron microscope, a mass spectrometry such as TOF-SIMS, or other methods. The thickness of the layer on the medical device surface of the present invention is preferably 100 $\mu$m or less, more preferably 10 $\mu$m or less, even more preferably 1 $\mu$m or less, and particularly preferably 0.5 $\mu$m or less because if it is excessively large, it is likely to be optically nonuniform. On the other hand, if the thickness of the layer is excessively small, the hydrophilicity of the surface tends to be insufficient; therefore, it is preferably 0.1 nm or more, more preferably 1 nm or more, even more preferably 10 nm or more, and particularly preferably 50 nm or more. Here, the thickness of the layer made of the hydrophilic polymer means the thickness in a dry state, and it can be determined by electron microscopy, or the like.

The layer containing the ionic polymers may further contain a nonionic polymer as a third component. In that case, from the viewpoint that good surface wettability and lubricity can be obtained, preferable examples of the hydrophilic polymer include polyvinylpyrrolidone, polyacrylamide, polydimethylacrylamide, poly(N-methylvinylacetamide), hydrophilic poly(meth)acrylate, polyalkylene glycol, polyvinyl alcohol, polyvinyl acetate, polyvinyl caprolactam, various cellulose derivatives, and various polysaccharides. Although the above are mainly examples of homopolymers, but copolymers thereof are also suitable.

In the present invention, when the coating layer is multilayered, it is sometimes difficult to substantially distinguish individual layers. Therefore, in one embodiment of the present invention, it is preferable that at least a part of the surface of the medical device substrate has a layer containing two or more ionic polymers and at least one of the two or more ionic polymers is a block polymer having a hydrophobic segment. The block polymer having a hydrophobic segment has a role of associating with a substrate via the hydrophobic segment using various interactions typified by hydrophobic interaction.

The layer containing two or more ionic polymers may contain two or more ionic polymers having like charges, but it is preferable in the present invention that the layer containing two or more ionic polymers each contain at least one ionic polymer having a positive charge and at least one ionic polymer having a negative charge because the durability of the coating layer is improved by their electrostatic interaction.

Of the polymers contained in the layer containing two or more ionic polymers, at least one polymer is particularly

preferably a block polymer having a hydrophobic segment.

In the present invention, the layer containing two or more ionic polymers preferably has one or more layers containing one or more ionic block polymers having the hydrophobic segment and one or more layers containing one or more ionic polymers having no hydrophobic segments.

If the layer containing two or more ionic polymers interact and are fixed at multiple points, the mobility of molecular chains is reduced and sufficient lubricity may not be obtained. Therefore, when a plurality of different types of ionic polymers are mixed seamlessly, it is sometimes difficult to obtain sufficient lubricity, and therefore the plurality of different types of ionic polymers preferably form separate layers. The layer containing two or more ionic polymers may be formed from only the ionic polymer having a hydrophobic segment, but if the hydrophobic segment is exposed on the surface of the layer, the biocompatibility of the outermost surface may be impaired. Therefore, the layer containing two or more ionic polymers preferably includes one or more layers containing one or more ionic block polymer having a hydrophobic segment and one or more layers containing one or more ionic polymer having no hydrophobic segment. The ionic block polymer having a hydrophobic segment and the ionic polymer having no hydrophobic segment may be present at any positions in the depth direction of the layer, but they are present preferably such that the hydrophobic segment is not exposed on the surface, and in order to increase interaction between the hydrophobic segment and the substrate and to make it easier to increase the durability of a coating layer, the ionic block polymer having a hydrophobic segment is preferably distributed unevenly in the vicinity of the substrate in the layered structure. It is also possible to form a covering layer of the ionic polymer having no hydrophobic segment and then associate the ionic block polymer having a hydrophobic segment to fill sites where the covering layer is incomplete. That is, although the ionic block polymer having a hydrophobic segment may be present in the vicinity of a surface in the layered structure, it is preferable in such a case that the hydrophobic segment is not exposed on the surface. Furthermore, the ionic block polymer having a hydrophobic segment may form a micelle-like structure on the surface of the substrate via the hydrophobic segment in the layered structure.

From the viewpoint of improving durability against delamination of the coating layer, it is more preferable that the one or more layers containing one or more ionic polymers having no hydrophobic segments contains an ionic polymer having a charge opposite to the ionic block polymer having a hydrophobic segment.

That is, in an embodiment of the present invention, a configuration capable of providing superior performance is a medical device having a layer containing at least one ionic block polymer having a hydrophobic segment on at least a part of its surface, wherein the medical device has thereon one or more layers containing an ionic polymer having a charge opposite to that of the ionic block polymer having a hydrophobic segment. A configuration in which a plurality of layers of an ionic polymer are further stacked alternately on the layer containing one or more ionic block polymers having a hydrophobic segment is preferable because particularly superior performance is obtained.

In a conventional method of forming a multilayered coating layer using only a highly hydrophilic ionic polymer, there are problems such as uneven coating and poor durability due to insufficient affinity with a substrate. In the present invention, however, it is believed that due to various interactions typified by hydrophobic interaction generated between a hydrophobic segment and a substrate, the affinity of a coating polymer with the substrate is enhanced, so that uneven coating hardly occur and the durability can be improved. However, as described above, an ionic block polymer having a hydrophobic segment used alone can only weakly bond to a medical device surface via interactions typified by hydrophobic interaction, so that it may easily delaminate. It is believed that by use of another polymer having no hydrophobic segment, especially, an ionic polymer having a charge opposite to that of the ionic block polymer having a hydrophobic segment in combination, a strong coating layer is formed on a medical device surface via various interactions typified by electrostatic interaction generated between these polymers, so that a coating layer having superior durability can be obtained.

In the present invention, in order to allow the interaction between the coating layer and the substrate to function efficiently, a configuration in which a layer containing an ionic block polymer having a hydrophobic segment is formed on at least a part of a surface of a medical device and a layer containing an ionic polymer is further formed thereon is more preferable.

In the present invention, it is preferable that the one or more layers containing one or more ionic polymers do not contain any ionic block polymer having a hydrophobic segment and are present as separate layers from a layer or layers containing an ionic block polymer having a hydrophobic segment. Such one or more layers containing one or more ionic polymers preferably contain an ionic polymer having a charge opposite to the ionic block polymer having a hydrophobic segment.

The ionic polymer having no hydrophobic segments preferably has a multi-layer structure composed of two or more types of ionic polymer. Such a multi-layer structure is preferable because good delamination resistance can be attained when a layer containing one or more polymers having a negative charge and one or more polymers having a positive charge is formed.

While it is believed that the block polymer having a hydrophobic segment can associate with a surface of a medical device substrate due to its affinity mainly based on hydrophobic interaction occurring between the hydrophobic

segment and the substrate, since the block polymer having a hydrophobic segment may not alone have sufficient delamination resistance, a coating layer with sufficient durability can be formed by using one or more other ionic polymers in combination and utilizing various interactions typified by electrostatic interaction between the ionic polymers and the block polymer having a hydrophobic segment and/or a medical device surface. The various interactions mentioned herein refer to entanglement (molecular entanglement of molecules), van der Waals force, dipole interaction, hydrophobic interaction), hydrogen bonding, $\pi$-$\pi$ stacking and combinations of these effects. Especially when the charge of a block polymer having a hydrophobic segment and the charge of another ionic polymer are opposite to each other, they are attracted by electrostatic interaction (Coulomb force) to form a polyion complex and become insoluble, so that the durability of a coating layer is improved. Further, it is preferable to stack a plurality of ionic polymers in layers to form a multilayer structure because durability is thereby improved to a practical level and biocompatibilities such as lubricity, water wettability, and antifouling property are also enhanced.

Therefore, in the present invention, it is preferable to use two or more different types of ionic polymers as the two or more ionic polymers. Even in the case of a combination of different types of ionic polymers having same-sign charges, for example, those capable of exhibiting such interaction as hydrogen bonding can preferably be used, but it is particularly preferable to use those having opposite charges from the viewpoint that relatively strong interaction is generated due to electrostatic interaction and superior durability can be obtained.

In the present invention, although the layer containing ionic polymers preferably has a multilayer structure composed of a plurality of ionic polymers, the layers may be mixed seamlessly and observed integrally and the layers may be substantially difficult to be recognized as multiple layers when the individual layers are thin or for the reason that the order of the layers are locally reversed.

Such a multilayer structure is preferably formed by performing one or more treatments with one or more negatively charged polymer solutions and one or more treatments with one or more positively charged polymer solutions.

The multilayer structure is formed on a surface of a substrate by performing the treatment with one or more negatively charged polymer solutions and the treatment with one or more positively charged polymer solutions, each, preferably once to 5 times, more preferably once to 3 times, even more preferably once to twice. The number of times of the treatment with a solution negatively charged solution may differ from the number of times the treatment with a positively charged polymer solution.

The treatment with a negatively charged polymer solution and the treatment with a positively charged polymer solution are preferably carried out alternately. It is further preferable to start the treatments with a polymer solution having a charge opposite to that of the ionic polymer having a hydrophobic segment.

In the medical device of the present invention, superior wettability and lubricity can be imparted by a very small number of times, namely, twice or three times in total, of treatment with one or more negatively charged polymer solutions and treatment with one or more positively charged polymer solutions. From the viewpoint of shortening the manufacturing process, this has a very important meaning in industrial aspect. In that sense, in the medical device of the present invention, it is preferable that the total number of the treatment with a negatively charged polymer solution and the treatment with a positively charged polymer solution is twice or three times.

The present inventors have confirmed that it is insufficient to develop wettability and lubricity merely by performing treatment only with either a negatively charged polymer solution or a positively charged polymer solution.

As the polymer having a positive charge, a homopolymer or a copolymerized polymer having a plurality of basic groups along the polymer chain can be suitably used. As a group having basic properties and affording a positive charge, an amino group and a salt thereof are preferable. For example, suitable examples of such a polymer having a positive charge include amino group-containing (meth)acrylate polymers such as poly(allylamine), poly(vinylamine), poly(ethyleneimine), poly(vinylbenzyltrimethylamine), polyaniline, poly(aminostyrene), poly(N,N-dialkylaminoethyl methacrylate), and amino group-containing (meth)acrylamide polymers such as poly(N,N-dimethylaminopropylacrylamide), and salts thereof. Although the above are example of homopolymers, mixtures of these polymers and copolymers with other monomers can also be suitably used.

In the present invention, the term "basic monomer" refers to a polymerizable monomer having a group having basic properties in its aqueous solution in pure water and affording a positive charge.

When the polymer having a positive charge is a copolymer, one containing a structure derived from a basic monomer that constitutes the copolymer can preferably be used. From the viewpoint of high degree of polymerizability, such a monomer is preferably a monomer having an allyl group, a vinyl group and a (meth)acryloyl group, and a monomer having a (meth)acryloyl group is particularly preferable. Examples of preferable monomers containing a group having basic properties to constitute a copolymer having a positive charge include allyamine derivatives, aminostyrene derivatives, amino group-containing (meth)acrylates such as N,N-dialkylaminoethyl methacrylate, amino group-containing (meth)acrylamindes such as N,N-dimethylaminopropylacrylamide, N-vinylimidazole derivatives and salts thereof. Among these, monomers containing a quaternary ammonium salt or an imidazolium salt are particularly preferable because they have antibacterial properties. Among these, from the viewpoint of high degree of polymerizability, amino group-containing (meth)acrylates, amino group-containing (meth)acrylamides, and salts thereof are more preferable, and N,N-dimethylaminoethyl methacrylate, N,N-dimethylaminopropylacrylamide, and salts thereof are particularly preferable.

As a polymer having a negative charge, a homopolymer or copolymer having a plurality of groups having acidic properties along the polymer chain can suitably be used. As the group having acidic properties and affording a negative charge, a carboxy group, a sulfo group (-$SO_3$H), a sulfuric acid group (-$OSO_3$H), a phosphonic acid group (-PO$(OH)_2$), a phosphoric acid group (-OPO$(OH)_2$) and salts thereof are suitable. Preferable examples of such polymers having a negative charge include polymethacrylic acid, polyacrylic acid, poly(vinylbenzoic acid), poly(thiophene-3-acetic acid), poly(4-styrenesulfonic acid), polyvinylsulfonic acid, poly(2-acrylamido-2-methylpropanesulfonic acid) and salts thereof. Although the above are example of homopolymers, mixtures of these polymers and copolymers with other monomers can also be suitably used.

In the present invention, the term "acidic monomer" refers to a polymerizable monomer having a group having acidic properties in its aqueous solution in neutral pure water and affording a negative charge.

When the polymer having a negative charge is a copolymer, one containing a structure derived from an acidic monomer that constitutes the copolymer can preferably be used. From the viewpoint of high degree of polymerizability, such a monomer is preferably a monomer having an allyl group, a vinyl group and a (meth)acryloyl group, and a monomer having a (meth)acryloyl group is particularly preferable. Examples of preferable acidic monomers to constitute the copolymer include (meth)acrylic acid, vinylbenzoic acid, styrenesulfonic acid, vinylsulfonic acid, 2-acrylamido-2-methylpropanesulfonic acid, and salts thereof. Among these, (meth)acrylic acid, 2-acrylamido-2-methylpropanesulfonic acid, phosphate-type (meth)acrylate monomers and their slats are more preferable, and (meth)acrylic acid and salts thereof are particularly preferable.

At least one of the polymer having a positive charge and the polymer having a negative charge to be used in the present invention is preferably a polymer having an amide structure and/or a hydroxyl group. It is more preferable that the polymer having a negative charge and/or the polymer having a positive charge has an amide structure because a surface superior in lubricity and water wettability can be obtained.

It is more preferable that a medical device has a layer containing at least one species selected from a polymer having a hydroxyl group and having a negative charge or a polymer having a hydroxyl group and a positive charge, or a polymer having an amide group and having a negative charge or a polymer having an amide group and having a positive charge on a surface of the device.

This case is preferable because there can be developed both an effect that a surface with lubricity is formed and an effect that a surface superior in antifouling property against biomolecule-derived molecules can be formed.

Examples of the polymer having an amide group and having a positive charge include polyamides having an amino group, partially hydrolyzed chitosan, and copolymers of basic monomers and monomers having an amide group.

Preferable specific examples of the copolymers of basic monomers and monomers having an amide group include N,N-dimethylaminoethyl methacrylate/N-vinylpyrrolidone copolymers, N,N-dimethylaminoethyl methacrylate/N,N-dimethylacrylamide copolymers, N,N-dimethylaminopropylacrylamide/N-vinylpyrrolidone copolymers, and N,N-dimethylaminopropylacrylamide/N,N-dimethylacrylamide copolymers. Particularly preferred are N,N-dimethylaminopropylacrylamide/N,N-dimethylacrylamide copolymers.

Examples of the polymer having an amide group and having a negative charge include polyamides having a carboxyl group and copolymers of acidic monomers and monomers having an amide group.

Preferable specific examples of the copolymers of acidic monomers and monomers having an amide group include (meth)acrylic acid/N-vinylpyrrolidone copolymers, (meth)acrylic acid/N,N-dimethylacrylamide copolymers, 2-acrylamide-2-methylpropanesulfonic acid/N-vinylpyrrolidone copolymers, and 2-acrylamide-2-methylpropanesulfonic acid/N,N-dimethylacrylamide copolymers. Particularly preferred are (meth)acrylic acid/N,N-dimethylacrylamide copolymers.

Examples of the polymers having a hydroxyl group and having a positive charge include aminopolysaccharides such as chitin and copolymers of basic monomers and monomers having a hydroxyl group.

Preferred specific examples of copolymers of a basic monomer and a monomer having a hydroxyl group include N,N-dimethylaminoethyl methacrylate/hydroxyethyl (meth)acrylate copolymers, N,N-dimethylaminoethyl methacrylate/glycerol (meth)acrylate copolymers, N,N-dimethylaminopropylacrylamide/hydroxyethyl (meth)acrylate, and N,N-dimethylaminopropylacrylamide/glycerol (meth)acrylate copolymers. Particularly preferred are N,N-dimethylaminoethyl methacrylate/hydroxyethyl (meth)acrylate copolymers.

Examples of the polymer having a hydroxyl group and having a negative charge include polysaccharides having an acidic group such as hyaluronic acid, chondroitin sulfate, carboxymethylcellulose, and carboxypropylcellulose, copolymers of an acidic monomer with a monomer having an amide group.

Specific examples of the copolymers of an acidic monomer with a monomer having a hydroxyl group include (meth)acrylic acid/hydroxyethyl(meth)acrylate copolymers, (meth)acrylic acid/glycerol(meth)acrylate copolymers, 2-acrylamido-2-methylpropanesulfonic acid/hydroxyethyl (meth)acrylate copolymers, and 2-acrylamido-2-methylpropanesulfonic acid/glycerol(meth)acrylate copolymers. Particularly preferred are (meth)acrylic acid/hydroxyethyl(meth)acrylate copolymers.

The monomer having an amide group to be used is not limited to the examples listed above and the monomers listed below as examples may also be used. In terms of ease of polymerization, a monomer having a (meth)acrylamide group and N-vinylcarboxylic acid amide (including a cyclic monomer) are preferable. Suitable examples of the monomer include N-vinylpyrrolidone, N-vinylcaprolactam, N-vinylacetamide, N-methyl-N-vinylacetamide, N-vinylformamide, N,N-dimethylacrylamide, N,N-diethylacrylamide, N-isopropylacrylamide, N-(2-hydroxyethyl)acrylamide, acryloylmorpholine, and acrylamide. Among these monomers, N-vinylpyrrolidone and N,N-dimethylacrylamide are preferable from the viewpoint of lubricity, and N,N-dimethylacrylamide is particularly preferable. Two or more members selected from these monomers may be used in combination.

The monomer having a hydroxyl group to be used is not limited to the examples listed above and the monomers listed below as examples may also be used. Preferred examples of the monomer include hydroxyethyl(meth)acrylate, hydroxypropyl(meth)acrylate, hydroxybutyl(meth)acrylate, hydroxyethyl(meth)acrylamide, glycerol(meth)acrylate, caprolactone-modified 2-hydroxyethyl(meth)acrylate, N-(4-hydroxyphenyl)maleimide, hydroxystyrene, and vinyl alcohol (carboxylic acid vinyl ester as a precursor). The monomer having a hydroxyl group is preferably a monomer having a (meth)acryloyl group from the viewpoint of ease of polymerization, and more preferably a (meth)acrylic acid ester monomer. Among these monomers, hydroxyethyl (meth)acrylate, hydroxypropyl(meth)acrylate and glycerol (meth)acrylate are preferable from the viewpoint of antifouling property against a lacrimal fluid, and hydroxyethyl(meth)acrylate is particularly preferable.

In the case of using the copolymer of a basic monomer or an acidic monomer with another monomer, the copolymerization ratio [the mass of the basic monomer or acidic monomer]/[the mass of the other monomer] is preferably 1/99 to 99/1, more preferably 2/98 to 90/10, and even more preferably 10/90 to 80/20. When the copolymerization ratio is within this range, functions such as lubricity and water wettability are easily exerted.

In order to change various characteristics, for example, thickness of the coating layer, it is possible to change the molecular weights of the polymer having a positive charge and the polymer having a negative charge. Specifically, when the molecular weight is increased, the thickness of the coating layer commonly increases. However, when the molecular weight is too large, it may become difficult to handle due to an increase in viscosity. Therefore, acidic polymers and basic polymers to be used in the present invention preferably have a molecular weight of 2,000 to 150,000. The molecular weight is more preferably from 5,000 to 100,000, and still more preferably from 75,000 to 100,000. The molecular weight of the acidic polymers and basic polymers is a polyethylene glycol-equivalent mass-average molecular weight measured by a gel permeation chromatographic method (aqueous solvent).

The application of the coating layer can be attained by many methods such as those disclosed in WO 99/35520, WO 01/57118, or US 2001/0045676 A1.

Here, there is described a method for forming a coating layer on at least a part of a surface of the medical device. It is preferable to form a coating layer in accordance with the procedures of the manufacturing method described below because mere use of the polymer having a hydrophobic segment and an ionic polymer in combination may not afford sufficient effects. By bringing a medical device substrate into contact with a solution of the polymer having a hydrophobic segment and a solution of the ionic polymer, a coating layer is formed on the medical device surface. As a method for bringing the medical device substrate into contact with the polymer solutions, various coating methods such as an immersion method (a dipping method), a brush coating method, a spray coating method, a spin coating method, a die coating method, and a squeegee method may be applied. Of these, the immersion method is preferred because it is simple.

From the viewpoint of shortening the manufacturing process, the method of manufacturing the medical device of the present invention is preferably a method of manufacturing a medical device including the following Steps 1a to 3a in this order. When it is not necessary to synthesize an ionic block polymer having a hydrophobic segment, for example, when an ionic block copolymer having a hydrophobic segment can be obtained by some method, the method of the present invention is a method of manufacturing the medical device of the present invention and preferably is a method including the following Steps 2a to 3a in this order:

<Step 1a>
the step of synthesizing an ionic block polymer having a hydrophobic segment;
<Step 2a>
the step of bringing at least a part of a medical device substrate into contact with a solution of an ionic block polymer having a hydrophobic segment and then removing a surplus polymer solution;
<Step 3a>
the step of bringing at least a part of the medical device substrate into contact with a solution of an ionic polymer and then removing a surplus polymer solution.

The ionic polymer in Step 3a preferably has a charge opposite to the ionic block polymer having a hydrophobic segment. It is believed that, as previously described, an ionic block polymer having a hydrophobic segment can associate

with a surface of a medical device due to the affinity of the polymer with the surface, and it is also believed that by bringing the ionic block polymer associated with the surface into contact with an ionic polymer solution having a charge opposite to the ionic block polymer having a hydrophobic segment, the layer is stabilized through their interaction and is improved in durability.

Steps 2a and 3a may be repeated in this order. This embodiment is preferable because a coating layer with a multi-layer structure will be formed and durability will be improved.

In Step 3a, a plurality of types of contact to the solution of the ionic polymer may be performed. In this case, it is preferable to perform the contact while selecting solutions of a plurality of ionic polymers so that their charges may alternate. Performing contact so that charges may alternate specifically means bringing a solution of a polymer having a positive charge and a solution of a polymer having a negative charge into contact alternately. The order of the positive charge and the negative charge may be reversed.

When the ionic block polymer having a hydrophobic segment has a positive charge, it is preferable to form a coating layer by performing the following Steps 1b to 3b in order:

<Step 1b> Step of bringing at least a part of a medical device substrate into contact with a solution containing an ionic block polymer having a hydrophobic segment (positive charge);
<Step 2b> Step of bringing a part of the medical device into contact with a solution containing an ionic polymer (negative charge), and then removing a surplus polymer; in order to enhance durability and biocompatibility, it is preferable to perform Step 3b subsequently after Step 2b,
<Step 3b> Step of bringing at least a part of the medical device into contact with a solution containing an ionic polymer (positive charge), and then removing a surplus polymer.

Moreover, Steps 2b and 3b may be repeated twice or more in order. This is preferable because a coating layer with a multi-layer structure will thereby be formed and therefore durability will be enhanced.

When the ionic block polymer having a hydrophobic segment has a negative charge, it is preferable to form a coating layer in the order of the following Step 1c to step 3c.

<Step 1c> Step of bringing at least a part of a medical device substrate into contact with a solution containing an ionic block polymer having a hydrophobic segment (negative charge);
<Step 2c> Step of bringing at least a part of the medical device into contact with a solution containing an ionic polymer (positive charge), and then removing a surplus polymer.
Subsequently, in order to improve durability and biocompatibility, it is preferable to carry out Step 3c after Step 2c.
<Step 3c> Step of bringing a part of the medical device into contact with a solution containing an ionic polymer (negative charge), and then removing a surplus polymer. Furthermore, Steps 2c and 3c may be repeated twice or more in this order. This is preferable because a coating layer with a multi-layer structure will thereby be formed and therefore durability will be enhanced.

When bringing the positively charged or negatively charged polymer solution into contact with a medical device substrate, the surface of the substrate may be untreated or may have been treated. Here, the surface of a substrate has been treated means that the surface of the substrate has been subjected to surface treatment or surface modification by a known technique. Suitable examples of the surface treatment or the surface modification include plasma treatment, excimer lamp irradiation treatment, chemical vapor deposition, chemical modification, etching, and plasma coating.

When the contact of the solution is performed by an immersion method, the immersion time may be varied according to many factors. Immersion of the molding in the polymer solution is carried out preferably for 1 to 30 minutes, more preferably for 2 to 20 minutes, and particularly preferably for 1 to 5 minutes.

In the present invention, the ionic polymer is preferably used as a solution in a protic solvent, more preferably in an alcohol or water. Water is particularly preferable from the viewpoint that it does not induce deformation of a substrate and has little adverse effects harmful to the human body.

The concentration of the polymer solution can be varied according to the properties of the ionic block polymer having a hydrophobic segment and the ionic polymer, the thickness of a desired coating layer, and many other factors. The preferred polymer concentration is 0.001 to 10% by mass, more preferably 0.005 to 5% by mass, even more preferably 0.01 to 3% by mass, and particularly preferably 0.7 to 1.3% by mass.

The pH of the polymer solution should be appropriately chosen so that the polymer may have a charge. An appropriate range varies depending on the properties of the polymer to be used, but in general, the pH is preferably kept within a range of 2 to 8, more preferably 3 to 7.

The thickness of the coating layer can be adjusted by adding one or more salts such as sodium chloride to the polymer solution. A preferable salt concentration is 0.1 to 2.0% by mass. As the salt concentration rises, the polyelectrolyte takes a more spherical three-dimensional structure. However, if the concentration becomes excessively high, the polyelectrolyte does not deposit well on a surface of a molding even if it deposits. A more preferable salt concentration is 0.7 to 1.3% by mass.

The removal of the surplus polymer solution by washing is commonly performed by rinsing with clean water or an organic solvent. Rinsing is preferably performed by immersing the molding in water or an organic solvent or exposing to a water flow or an organic solvent flow. Rinsing may be completed in one step. However, it was recognized that it is more efficient that a rinsing step is performed twice or more. Rinsing is preferably performed in 2 to 5 steps. Immersion of each molding in a rinsing solution is preferably performed for 1 to 3 minutes. Pure water is preferably used as the rinsing solution. In order to increase adhesion of a coating layer, it is preferred to use an aqueous buffered solution having pH adjusted within a range from 2 to 7, more preferably from 2 to 5, and even more preferably from 2.5 to 4.5.

The step of drying or removing an excess rinsing solution may also be included. Although a molding can be dried to some extent by merely leaving the molding to stand under air atmosphere, drying can be accelerated by supplying a mild air flow to the surface. The flow rate of the air flow can be adjusted as a function of the strength of a material to be dried, and mechanical fixing of a material. There is no need to completely dry the molding. Herein, it is important to remove droplets of a solution adhered onto a surface of the molding as compared with drying of the molding. Therefore, the molding is only dried until a film of water or a solution on the surface of the molding is removed, preferably leading to shortening of the process time.

The solution of the ionic block polymer having a hydrophobic segment is preferably brought into contact with the medical device before the solution containing the other ionic polymer, but it is also allowable to form a layer from the other ionic polymer and then bring the solution of the ionic block polymer having a hydrophobic segment into contact in order to fill the hole where coating has been attained insufficiently.

Next, taking an ophthalmic lens, especially a contact lens, which is a particularly preferable medical device in the present invention, as an example, a method for the manufacture thereof will be described. In the present invention, an ophthalmic lens is manufactured by bringing a solution of one or more block polymers having a hydrophobic segment into contact with at least a part of the surface of a lens-shaped molding (substrate) and also bringing a solution of one or more ionic polymers into contact. The number of times of the contact with each solution is preferably 1 to 5 times, more preferably 1 to 3 times, and even more preferably 1 to 2 times. The number of times of application of the solution of the block polymer having a hydrophobic segment and that of the solution of the ionic polymer may be different.

In the present invention, one preferred embodiment of the method of manufacturing an ophthalmic lens includes the following Steps 1d to 4d in this order.

<Step 1d>
the step of polymerizing a mixture of monomers to obtain a lens-shaped molding;
<Step 2d>
the step of bringing the molding into contact with a solution of an ionic block polymer having a hydrophobic segment and then removing a surplus polymer solution by washing,
<Step 3d>
the step of bringing the molding into contact with an ionic polymer solution having a charge opposite to that of the ionic block polymer having a hydrophobic segment and then removing a surplus polymer solution by washing;
<Step 4d>
the step of bringing the molding into contact with a solution of an ionic polymer having a charge like that of the ionic block polymer having a hydrophobic segment and then removing a surplus polymer solution by washing;
Steps 3d and 4d may be repeated a plurality of times in this order.

In addition, a coating layer can be formed on a lens-shaped molding by sequentially bringing the molding into contact with a solution of an ionic block polymer having a hydrophobic segment and a solution of an ionic polymer. After that, it is preferable to remove surplus polymers by washing sufficiently.

When the medical device of the present invention is used as an ophthalmic lens, especially as a contact lens, the polymerization method and the molding method of the substrate may be standard methods like those described below. Examples thereof include a method of finishing a polymer shaped in a round rod form or a plate form into a desired shape by cutting or lathe processing, a mold polymerization method, and a spin casting method.

The medical device substrate in the present invention preferably contains silicon atoms in at least a part thereof in order to obtain good affinity with the block polymer having a hydrophobic segment in the present invention. Specifically, the substrate contains 1% by mass or more of silicon atoms. The silicon atom content (% by mass) is calculated on a basis (100% by mass) of the mass of the substrate in a dry state. The silicon atom content of the substrate is more preferably 2% by mass or more, even more preferably 5% by mass or more, particularly preferably 7% by mass or more, and most preferably 10% by mass or more. The silicon atom content of the substrate is more preferably 70% by mass or less, even more preferably 60% by mass or less, and particularly preferably 50% by mass or less because an excessively high content of silicon atoms may lead to a high tensile modulus and therefore may be undesirable. Especially

when the medical device is to be used for contact lens application, in order to make the medical device not too hard, the silicon atom content is preferably 36% by mass or less, more preferably 30% by mass or less, and even more preferably 26% by mass or less. Any of the upper limits may be combined with any of the lower limits. The silicon atoms may be present in the form of siloxanyl groups. Compounds formed by polymerizing monomers having a siloxanyl group and a radically polymerizable functional group, such as a (meth)acryloyl group, a (meth)acryloyloxy group, or a (meth)acrylamide group, at an end thereof can suitably be used, and examples thereof include siloxane compounds with a polydimethylsiloxane structure having methacryloyloxy groups at both ends. It is also possible to mix a hydrophilic compound, e.g., (meth)acrylamides such as N,N-dimethylacrylamide, N-vinylamides such as N-vinylpyrrolidone, hydroxyalkyl(meth)acrylates such as 2-hydroxyethyl(meth)acrylate and 2-methoxyethyl (meth)acrylate, and alkyl ethers thereof, polyethylene glycol mono(meth)acrylates such as diethylene glycol mono(meth)acrylate and diethylene glycol mono(meth)acrylate methyl ether, and methyl ethers thereof, and optionally crosslinkable compounds, e.g., di(meth)acrylates such as polyethylene glycol di(meth)acrylate, N,N-methylene bisacrylamide, and polyfunctional (meth)acrylates, with such a silicon atom-containing compound, obtain a copolymer, and then form a substrate. When making a medical device have a low water content, it is possible to mix an alkyl (meth)acrylate, such as butyl acrylate and ethylhexyl acrylate, with such a silicon atom-containing compound, obtain a copolymer, and form a substrate.

When the medical device of the present invention is a contact lens, the medical device substrate can be any of a high water content lens, a low water content lens, and a non-hydrous lens. From the viewpoint that high affinity with an ionic block polymer having a hydrophobic segment can be obtained, a low water content lens and a non-hydrous lens are preferable. The "low water content lens" as referred to in the present invention means a lens having a water content of less than 50%, and the "high water content lens" means a lens having a water content of 50% or more.

The upper limit of the water content of the medical device substrate of the present invention is preferably 60% or less, more preferably 50% or less, and particularly preferably 40% or less from the viewpoint that it is possible to obtain high affinity with an ionic block polymer having a hydrophobic segment. The ionic block polymer having a hydrophobic segment can preferably be used also for a non-hydrous lens. The lower limit of the water content is preferably 0%, more preferably 0.1%, and particularly preferably 0.2%. Any of the upper limits may be combined with any of the lower limits.

The water content referred to in the present invention can be determined by measuring the mass of a lens with a surface from which surplus water has been wiped off after 8 hours or more immersion in a phosphate buffer (i.e., the mass in a wet state: W1) and the mass of the lens after being heated at 40°C in a vacuum dryer and concurrently dried for 8 hours or more (i.e., the mass in a dry state: W2), and then calculating a water content from the following formula:

$$\text{Water content (\%)} = (W1 - W2) / W1 \times 100$$

In the present invention, when the medical device is a soft contact lens, the tensile elastic modulus thereof is preferably 0.01 MPa or more and more preferably 0.1 MPa or more, and is preferably 5 MPa or less, more preferably 3 MPa or less, even more preferably 2 MPa or less, still even more preferably 1 MPa or less, and particularly preferably 0.6 MPa or less. If the tensile elastic modulus is excessively small, the device may be difficult to handle because it is excessively soft. If the tensile elastic modulus is excessively large, the device may have decreased wearing feeling because it is excessively hard. It is preferable that the tensile elastic modulus is 2 MPa or less because satisfactory wearing feeling is obtained, and it is preferable that the tensile elastic modulus is 1 MPa or less because more favorable wearing feeling is obtained. The tensile elastic modulus is measured using a specimen in a wet state with a borate buffer.

When the medical device is shaped by polymerization, various organic or inorganic solvents are applicable as a polymerization solvent. Examples thereof include water, alcohol solvents such as methanol, ethanol, propanol, 2-propanol, butanol, tert-butanol, tert-amyl alcohol, and 3,7-dimethyl-3-octanol; aromatic hydrocarbon-solvents such as benzene, toluene, and xylene; aliphatic hydrocarbon-solvents such as hexane, heptane, octane, decane, petroleum ether, kerosene, ligroin, and paraffin; ketone solvents such as acetone, methyl ethyl ketone, and methyl isobutyl ketone; ester solvents such as ethyl acetate, butyl acetate, methyl benzoate, dioctyl phthalate, and ethylene glycol diacetate; and glycol ether solvents such as diethyl ether, tetrahydrofuran, dioxane, ethylene glycol dialkyl ether, diethylene glycol dialkyl ether, triethylene glycol dialkyl ether, tetraethylene glycol dialkyl ether, polyethylene glycol dialkyl ether, polyethylene glycol-polypropylene glycol block copolymers, and polyethylene glycol-polypropylene glycol random copolymers. These solvents may be used singly or in combination. Of these, water, tert-butanol, tert-amyl alcohol, and 3,7-dimethyl-3-octanol are more preferable in resisting inhibiting radical polymerization.

The monomer concentration in a polymerization stock solution in the case of using a polymerization solvent is preferably 10% by mass to 80% by mass, more preferably 15% by mass to 65% by mass, and particularly preferably 20% by mass to 50% by mass because if the concentration is excessively low, a sufficient molecular weight is not obtained, and if the concentration is excessively high, this leads to an increased risk of runaway due to polymerization heat.

The medical device of the present invention preferably has lubricity on the surface of the medical device from the viewpoint of affinity of the device to living bodies and smoothening movement of the device when being in contact with the surface of a body tissue and the viewpoint of preventing adhesion to the cornea of wearers especially when the embodiment of the medical device of the present invention is a contact lens.
The lubricity can be evaluated by sensory evaluation when rubbing five times with a human finger. Samples having higher scores in the evaluation by the method disclosed in examples of the present description are preferred. For example, five-stage sensory evaluation in accordance with the following criteria can be performed, and score 2 or more is preferable, 3 or more is more preferable, 4 or more is even more preferable, and 5 is particularly preferable.

5: Excellent lubricity
4: Intermediate lubricity between 5 and 3
3: Moderate lubricity
2: Slight lubricity (intermediate lubricity between 3 and 1)
1: No lubricity

The medical device of the present invention preferably has a coating layer having sufficient delamination resistance (durability) against scrubbing. The durability of a coating layer can be evaluated based on the degree of resistance of score to drop by scrubbing with a contact lens cleaning solution a prescribed times and then performing sensory evaluation of the lubricity. The coating layer preferably has a lubricity of score 2 or more even after scrubbing four times, more preferably has a lubricity of score 2 or more even after scrubbing seven times, and particularly preferably has a lubricity of score 2 or more even after scrubbing fourteen times.
The medical device of the present invention preferably has sufficient water wettability. The water wettability can be evaluated by measuring a static water contact angle of a medical device surface.
The medical device of the present invention preferably has a static water contact angle of 0 degrees or more and 100 degrees or less, more preferably 0 degrees or more and 90 degrees or less, even more preferably 0 degrees or more and 80 degrees or less, and particularly preferably 0 degrees or more and 70 degrees or less.
For example, when the embodiment of the present invention is a contact lens, from the viewpoint of preventing adhesion to the cornea of wearers, the static contact angle is preferred to be smaller, and is preferably 65 degrees or less, more preferably 60 degrees or less, even more preferably 55 degrees or less, still even more preferably 50 degrees or less, and particularly preferably 45 degrees or less. The static contact angle is measured against a phosphate buffer or RO water using a sample wetted with a phosphate buffer.
The medical device of the present invention preferably has antifouling property, namely adhesion resistance, against biomolecules. Causes of a stain on medical devices include adhesion of proteins and lipids. The adhesion resistance against proteins and lipids can be evaluated by mucin adhesion and lipid (methyl palmitate) adhesion. The smaller the adhesion amount measured by these evaluations, the better the biocompatibility and the less the risk of bacterial proliferation, which is preferable.
When the embodiment of the present invention is a contact lens that contains a silicone material, adhesion of lipids may be a more serious problem than adhesion of proteins. Adhesion resistance against lipids can be evaluated by the method described in the working examples of the present description using methyl palmitate as a model molecule of lipids. The evaluation can be performed by visually observing white turbidity of a sample and then determining the amount of methyl palmitate adhered to the sample according to the following criteria. According to the following criteria, 3 or more is preferable, 4 or more is more preferable, and 5 is particularly preferable.

5: Transparent with no white turbidity
4: Most part has no white turbidity
3: Almost half part has no white turbidity
2: Slight part has no white turbidity
1: White turbidity accounts for entirety

EXAMPLES

Examples of the present invention are hereafter explained by reference to examples, but the present invention is not limited by these examples.

<Analysis Method and Evaluation Method>

(1) Measurement of molecular weight of ionic block polymer having hydrophobic segment

Measurement was performed using a Prominence GPC system manufactured by Shimadzu Corporation. The device configuration is as follows. Pump: LC-20AD, autosampler: SIL-20AHT, column oven: CTO-20A, detector: RID-10A, column: GMPWXL manufactured by Tosoh Corporation (inner diameter 7.8 mm × 30 cm, particle diameter 13 μm). Measurement was carried out using water/methanol = 1/1 (with addition of 0.1N lithium nitrate) as an eluent at a flow rate of 0.5 mL/min and a measurement time of 30 minutes. The sample concentration was adjusted to 0.2% by mass, and the sample injection amount was adjusted to 100 μL. A working curve was produced using polyethylene oxide standard samples (0.1 kD to 1258 kD) manufactured by Agilent.

In the present description, the number-average molecular weight refers to a polyoxyethylene-equivalent number-average molecular weight measured by gel permeation chromatography (GPC) using a mixed solution of water : methanol = 1 : 1 containing 0.5N lithium nitrate as a solvent. The mass-average molecular weight and the polydispersity index (the value obtained by dividing the mass-average molecular weight by the number-average molecular weight) are also measured in the same manner as above.

In the present description, the mass-average molecular weight may be represented by Mw and the number-average molecular weight may be represented by Mn. In addition, a molecular weight of 1000 may be expressed by 1 kD. For example, the expression "Mw 33 kD" means "a mass-average molecular weight of 33000."

(2) Lubricity and scrubbing durability thereof

A contact lens-shaped specimen was immersed in a phosphate buffer contained in a vial at room temperature and was steam sterilized. The specimen was pulled out of the phosphate buffer and was subjected to sensory evaluation according to the following five-stage evaluation after rubbing the sample five times with a human finger, and a lubricity at 0 cycles was adopted.

Moreover, the sample subjected to the above evaluation was placed in a recess formed in the center of a palm of a hand and a cleaning solution ("OPTI FREE" (registered trademark) produced by Alcon Japan Ltd.) was added. Then, the sample was rubbed ten times on the front and back sides thereof with a ball of the forefinger of the other hand and then was washed well with water. The above series of operations was defined as one cycle, and 14 cycles were repeated. Thereafter, the sample was washed with pure water and then immersed in a phosphate buffer. Sensory evaluations at the 1 cycle, the 7 cycle, and the 14 cycle were carried out according to the following five-stage evaluation. The evaluation results at the 0, 1, 7, and 14 cycles are shown in Table 1.

5: Excellent lubricity
4: Intermediate lubricity between 5 and 3
3: Moderate lubricity
2: Little lubricity (intermediate lubricity between 3 and 1)
1: No lubricity

(3) Resistance to adhesion of lipid (Evaluation of antifouling property)

A stirrer (36 mm) was placed in a 500 ml beaker, and 1.0 g of methyl palmitate and 500 g of pure water were charged. The temperature of a water bath was set at 37°C and the above beaker was placed in the center of the water bath, followed by stirring for one hour using a magnetic stirrer. The rotation speed was set at 760 rpm. Spherical crown-shaped samples (edge diameter of about 14 mm, thickness of about 0.1 mm) were placed one by one in a lens basket and then placed in the beaker, followed by stirring. After one hour, stirring was stopped and the samples in the lens basket were scrubbed with tap water at 37°C and a liquid detergent for domestic use ("Mamalemon" (registered trademark), manufactured by Lion Corporation). The washed samples were placed in a 12-well plastic dish containing distilled water and allowed to stand overnight in a refrigerator. Then, white turbidity of the samples was visually observed and the amount of methyl palmitate adhered to the samples was judged according to the following criteria.

5: Transparent with no white turbidity
4: Most part has no white turbidity
3: Almost half part has no white turbidity
2: Slight part has no white turbidity
1: White turbidity accounts for entirety

(4) Static contact angle of water (Evaluation of water wettability)

A static contact angle was measured by a liquid drop method using WET 6000 manufactured by KYOWA. A contact lens substrate and a surface treated lens substrate were immersed in RO water for 24 hours or more. About 1DL of liquid drop (RO water) was brought into contact with each of the substrates using a microsyringe and the angle at which a lens surface and the liquid drop are in contact was measured as a contact angle.

(5) Atomic force microscope (AFM)

AFM was used to observe the form of a contact lens surface. Using WET-SPM9500J3 manufactured by Shimadzu Corporation, height images (a range of 0 to 50 nm) within a range of 5 μm square were measured in phase mode in the air.

[Preparation Example 1]

<Preparation of substrate>

The following components were mixed as component a and were stirred well.
Trifluoroethyl acrylate (Viscoat 3F, Osaka Organic Chemical Industry Ltd.) (57.9 parts by mass)
2-Ethylhexyl acrylate (7 parts by mass)
Dimethylaminoethyl acrylate (0.1 parts by mass) Ultraviolet absorber having a polymerizable group (RUVA-93, Otsuka Chemical Co., Ltd.) (0.5 parts by mass)
Colorant Reactive Blue 246 (0.02 parts by mass) Polymerization initiator "Irgacure" (registered trademark) 819 (Ciba Specialty Chemicals, 0.5 parts by mass)
t-Amyl alcohol (10 parts by mass)

Polydimethylsiloxane having methacryloyloxy groups at both ends (FM 7726, JNC, mass-average molecular weight: 29 kD, number-average molecular weight: 26 kD) (28 parts by mass) as a component b and polydimethylsiloxane having a methacryloyloxy group at one end (FM 0721, JNC, mass-average molecular weight: 5000) (7 parts by mass) were added to the mixed solution of the component a, and the mixture was well mixed and stirred.

This mixture was filtered through a membrane filter (0.45 μm) to remove insolubles to obtain a polymerization stock solution. This polymerized stock solution was poured into a contact lens mold made of transparent resin (base curve side: polypropylene, front curve side: ZEONOR), and was polymerized by light irradiation (1.01 $mW/cm^2$, 20 minutes) using fluorescent lamps (manufactured by TOSHIBA CORPORATION, FL 6D, daylight color, 6 W, four lamps). After the polymerization, the product contained in the mold was immersed in isopropyl alcohol and was heated at 80°C for one hour, and thus a contact lens-shaped molding was removed from the mold. The resulting molding was immersed in isopropyl alcohol at room temperature for 30 minutes, and then was dried with the molding being contained in a clean plastic container lined with mesh made of "Teflon (trademark)." The resulting lens substrate had an edge portion having a diameter of about 14 mm and had a thickness of about 0.07 mm at the center thereof.

[Synthesis Example 1]

<Synthesis example of ionic block polymer having hydrophobic segment>

A polysiloxane-containing macroinitiator was synthesized according to the method described in Example 3 (paragraphs 0038 to 0041) of JP-A-2012-246489. A 300 mL three-neck flask was charged with N,N-dimethylacrylamide (DMA, manufactured by Wako Pure Chemical Industries, Ltd., 4.372 g, 44.1 mmol), acrylic acid (AA, manufactured by Wako Pure Chemical Industries, Ltd., 0.353 g, 4.9 mmol), the polysiloxane-containing macroinitiator (12.3 mg, 49 μmol), and t-amyl alcohol (TAA, manufactured by Tokyo Chemical Industry Co., Ltd., 20.1 g), and a digital thermometer, a cooling tube with a three-way cock, and a sealer with stirring blades were fitted. Under irradiation with ultrasonic waves, a cycle including sucking to 10 mmHg and nitrogen flushing was repeated about five times to remove oxygen dissolved in the mixed solution. Subsequently, the mixture was allowed to react for 1 hour with stirring on an oil bath at 70°C, then heated to 75°C and reacted for five hours. After confirming that the viscosity had increased, the reaction vessel was pulled out of the oil bath and was allowed to cool. To the polymerization reaction solution, 50 mL of ethanol was added and the mixture was stirred to reduce viscosity, and then the mixture was poured into 550 mL of hexane to precipitate a polymer. The precipitated polymer was redissolved in 40 mL of ethanol and reprecipitated by pouring 360 mL of hexane. After repeating the same operation twice, the resultant was heated and dried in a vacuum dryer at 40°C overnight. The dried polymer was frozen and pulverized using liquid nitrogen to obtain a powder, and dried again in the vacuum dryer. The amount of the resulting polymer powder was 3.183 g and the powder had a number-average molecular weight (Mn)

of 153,000 and a mass-average molecular weight (Mw) of 446,000, and the desired product (P1) was obtained in a yield of 78.6%.

[Synthesis Example 2]

A desired product (P2) was synthesized in the same manner as in Synthesis Example 1 except that acrylic acid was replaced by 2-acrylamido-2-methylpropanesulfonic acid (AMPS, manufactured by Wako Pure Chemical Industries, Ltd.).

[Synthesis Example 3]

A desired product (P3) was synthesized in the same manner as in Synthesis Example 1 except that N,N-dimethylaminoethyl acrylate (DMAEA, produced by Tokyo Chemical Industry Co., Ltd.) was used instead of acrylic acid.

[Synthesis Example 4]

A desired product (P4) was synthesized in the same manner as in Synthesis Example 1 except that N,N-dimethylaminopropyl acrylamide methyl chloride quaternary salt (DMAPAA-Q, manufactured by Kj Chemicals Corporation) was used instead of acrylic acid.

[Synthesis Example 5]

A desired product (P5) was synthesized in the same manner as in Synthesis Example 1 except that the monomer used was replaced by N-vinylpyrrolidone. For the synthesis, reference was made to JP-A-2014-514422.

The number-average molecular weight (Mn), mass-average molecular weight (Mw), and polydispersity index (PDI) of the block copolymers obtained in Synthesis Examples 1 to 5 were as shown in Table 1.

[Table 1]

| | Name of polymer | Mn (kg/mol) | Mw (kg/mol) | PDI* |
|---|---|---|---|---|
| Synthesis Example 1 | P1 | 153 | 446 | 2.9 |
| Synthesis Example 2 | P2 | 59 | 210 | 3.5 |
| Synthesis Example 3 | P3 | 36 | 127 | 3.6 |
| Synthesis Example 4 | P4 | 185 | 535 | 2.9 |
| Synthesis Example 5 | P5 | 61 | 230 | 4.3 |
| * In Table 1, PDI: Poly Dispersity Index, (PDI = Mw/Mn) | | | | |

[Example 1]

<Preparation of surface treating liquid>

As surface treating liquids for medical devices, 1% by mass aqueous solutions of P1 to P5 were prepared. Such aqueous solutions of polymers were used after being through a filter. Likewise, 1% by mass aqueous solutions of polyacrylic acid (PAA, manufactured by Toagosei Co., Ltd., 250 kDa), polyethyleneimine (PEI, manufactured by Junsei Chemical Co., Ltd., 750 kDa), and poly(dimethylacrylamide-co-acrylic acid) (hereinafter CPDA, manufactured in a laboratory, the polymer disclosed in Synthesis Example 7 of WO 2011/102356), which were then used after being filtered with a filter having a 0.45 $\mu$m pore size.

<Coating>

A lens substrate was immersed in a 1% by mass aqueous solution of P1 and was left to stand at room temperature for 30 minutes, and then the lens substrate was removed from the aqueous P1 solution and was rinsed quickly with RO water three times.

Subsequently, the lens substrate was immersed in a 1% by mass aqueous solution of PEI and was left to stand at room temperature for 30 minutes, and then the lens substrate was removed from the aqueous PEI solution and was

rinsed quickly with RO water three times.

Subsequently, the lens substrate was immersed in a 0.1% by mass aqueous solution of CPDA and was left to stand at room temperature for 30 minutes, and then the lens substrate was removed from the aqueous CPDA solution and was rinsed quickly with RO water three times.

Surplus water was removed from the rinsed lens, which was then immersed in a phosphate buffer contained in a vial and steam sterilized (121°C, 30 minutes) to examine the effect of surface treatment.

The observation with an AFM of the surface of the coated lens obtained in Example 1 revealed that the surface was relatively smooth and suggested that there was less unevenness of coating. Fig. 1 is an AFM observed image (1) of the surface of the coated lens obtained in Example 1, and the surface was confirmed to be a smooth surface in which the area of hills colored white was large and almost no valleys colored black were found.

[Examples 2 to 9]

Samples were prepared in the same manner as in Example 1 except that the order of aqueous polymer solutions to be used for surface treatment was changed as shown in Table 2.

[Table 2-1]

| | Treatment procedure | | | |
|---|---|---|---|---|
| | 1 | 2 | 3 | 4 |
| Example 1 | P1 | PEI (+) | CPDA | |
| Example 2 | P2 | PEI (+) | CPDA | |
| Example 3 | P3 (+) | CPDA | | |
| Example 4 | P4 (+) | CPDA | | |
| Example 5 | P3 (+) | PAA | PEI (+) | CPDA |
| Example 6 | P4 (+) | PAA | PEI (+) | CPDA |
| Example 7 | P1 | P4 (+) | CPDA | |
| Example 8 | PAA | PEI (+) | P1 | |
| Example 9 | P1 | CPDA | | |
| Comparative Example 1 | P5 | | | |
| Comparative Example 2 | PAA | PEI (+) | CPDA | |
| Comparative Example 3 | P1 | | | |
| In Table 2-1, (+) indicates a polymer having a positive charge. | | | | |

[Table 2-2]

| | Lubricity after scrubbing | | | | | Water wettability (°) | Antifouling property Bad = 1... Good = 5 |
|---|---|---|---|---|---|---|---|
| | 0 times | Once | 4 times | 7 times | 14 times | | |
| Example 1 | 5 | 5 | 4 | 3 | 2 | 48.9 | 3 |
| Example 2 | 5 | 5 | 4 | 3 | 2 | 42.6 | 3 |
| Example 3 | 5 | 4 | 3 | 2 | 1 | 94.1 | 4 |
| Example 4 | 5 | 4 | 2 | 1 | 1 | 98.4 | 5 |
| Example 5 | 5 | 5 | 4 | 3 | 2 | 55.3 | 4 |
| Example 6 | 5 | 5 | 4 | 3 | 2 | 72.3 | 4 |
| Example 7 | 4 | 3 | 2 | 1 | 1 | 94.9 | 5 |
| Example 8 | 4 | 4 | 3 | 2 | 2 | 54.9 | 3 |

(continued)

| | Lubricity after scrubbing | | | | | Water wettability (°) | Antifouling property Bad = 1... Good = 5 |
|---|---|---|---|---|---|---|---|
| | 0 times | Once | 4 times | 7 times | 14 times | | |
| Example 9 | 4 | 3 | 2 | 1 | 1 | 98.4 | 3 |
| Comparative Example 1 | 2 | 1 | 1 | 1 | 1 | 102.0 | 1 |
| Comparative Example 2 | 4 | 3 | 1 | 1 | 1 | 62.6 | 2 |
| Comparative Example 3 | 4 | 3 | 1 | 1 | 1 | 97.8 | 2 |

[Comparative Example 1]

Comparative Example 1 is directed to a wetting agent having a siloxane segment at an end disclosed in Patent Documents 3 and 4. The lens substrate prepared in Preparation Example 1 was immersed in a 1% by mass aqueous solution of the P5 polymer prepared in Synthesis Example 5 for 30 minutes and then was rinsed with RO water three times and steam sterilized (121°C, 30 minutes) to evaluate in the prescribed items. The lubricity of the lens treated with the P5 polymer failed to last long and delamination readily occurred, that is, the durability was poor, and the antifouling property was also insufficient.

[Comparative Example 2]

Comparative Example 2 is directed to the surface coating disclosed in Patent Documents 1 and 2. In accordance with Example 19 of WO 2011/102356, the substrate prepared in Preparation Example 1 was surface treated by performing immersion and rinsing in order with aqueous solutions of PAA, PEI, and CPDA and then was steam sterilized (121°C, 30 minutes) to evaluate in the prescribed items. The water wettability and the lubricity were good, but the durability of lubricity was insufficient and the antifouling property was also insufficient. An observation with AFM confirmed that the surface of the coated lens obtained in Comparative Example 2 was a surface with many valleys and suggested that there was more unevenness in coating as compared with Example 1. Fig. 2 is an AFM observed image (2) of the surface of the coated lens obtained in Comparative Example 2, in which parts colored white are hills and parts colored black are valleys, and the surface was confirmed to be a rugged uneven surface.

[Comparative Example 3]

Comparative Example 3 is the case of immersion in a 1% by mass solution of an ionic block polymer having a hydrophobic segment for 30 minutes followed by rinsing with RO water and subsequent steam sterilization (121°C, 30 minutes). The sample had relatively good lubricity in the early stage, but the lubricity failed to last long as in Comparative Example 1 and sufficient antifouling property was not attained. From this result, it was confirmed that if the hydrophilic polymer having a hydrophobic segment disclosed in Patent Document 3 or 4 is used singly, it merely is held by a hydrophobic interaction and the effect does not last long. In addition, it was also confirmed that the water wettability is prone to undesirably drop because hydrophobic segments possibly appear on the surface and, therefore, the product is difficult to be used as a coating material.

## Claims

1. A medical device having a layer containing two or more ionic polymers on at least a part of a surface of a medical device substrate, wherein at least one of the two or more ionic polymers is a block polymer having a hydrophobic segment.

2. The medical device according to claim 1, wherein the layer containing two or more ionic polymers contains one or more ionic polymers having a positive charge and one or more ionic polymers having a negative charge.

3. The medical device according to claim 1, wherein the layer containing two or more ionic polymers includes one or more layers containing one or more ionic block polymers having the hydrophobic segment and one or more layers containing one or more ionic polymers having no hydrophobic segment.

4. The medical device according to claim 3, wherein the one or more layers containing one or more ionic polymers having no hydrophobic segment contains an ionic polymer having a charge opposite to that of the ionic block polymer having a hydrophobic segment

5. The medical device according to any one of claims 1 to 4, wherein a mass-average molecular weight of the block polymer having a hydrophobic segment is within a range of 10,000 to 10,000,000.

6. The medical device according to any one of claims 1 to 5, wherein the hydrophobic segment contains a polydimethylsiloxane structure.

7. The medical device according to any one of claims 1 to 6, wherein a structure of the block polymer having the hydrophobic segment is represented by the formula (b1):

[Chemical formula 1]

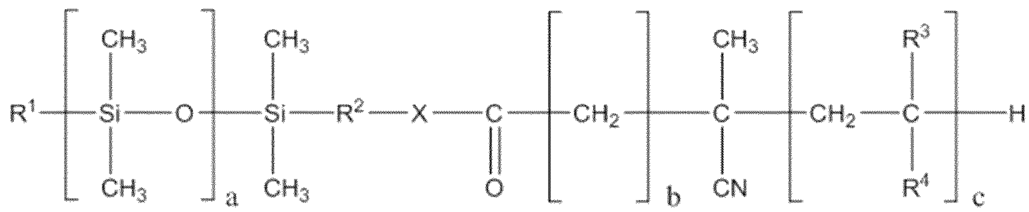

(b1)

in the formula (b1), $R^1$ is an alkyl group or an alkoxy group, $R^2$ is $(CH_2)_n$ or $(CH_2)_m$-$O(CH_2)_n$, m and n are each independently an integer of 1 to 16, a is 4 to 19, b is 1 to 6, c is 1 to 10000, X is O, NH, or S, $R^3$ represents H or $CH_3$, $R^4$ contains one or more structures represented by the formulae (a1) to (a3):

[Chemical formula 2]

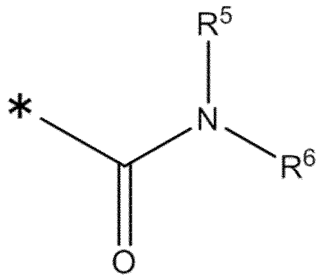

(a 1)

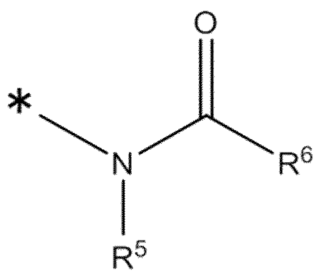

(a 2)

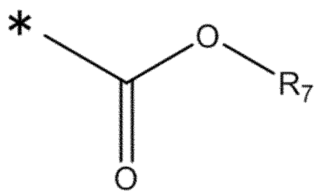

(a 3)

in the formulae (a1) to (a3), each * represents a bonding site to a polymer backbone; in the formulae (a1) to (a3), $R^5$ to $R^7$ are each independently a hydrogen atom or an alkyl group having 1 to 20 carbon atoms that may be branched or linear and optionally has a cyclic structure and optionally is substituted; $R^5$ and $R^6$ may form a ring via a bond.

8. The medical device according to any one of claims 1 to 7, wherein the block polymer having the hydrophobic segment contains 0.01 to 10% by mass of the hydrophobic segment and further contains 90 to 99.9% by mass of a hydrophilic segment.

9. The medical device according to any one of claims 1 to 8, wherein repeating units other than hydrophobic segments in the block polymer having the hydrophobic segment include at least one amide structure.

10. The medical device according to any one of claims 1 to 9, wherein the block polymer having the hydrophobic segment contains at least one of a structure derived from N-vinylpyrrolidone or a structure derived from N,N-dimethylacrylamide.

11. The medical device according to any one of claims 1 to 10, which is an ophthalmic lens.

12. The medical device according to claim 11, wherein the ophthalmic lens is a contact lens.

13. A method of manufacturing a medical device according to any one of claims 1 to 12, the method comprising the following Steps 2a to 3a in this order:

<Step 2a>
the step of bringing at least a part of a medical device substrate into contact with a solution of an ionic block polymer having a hydrophobic segment and then removing a surplus polymer solution;
<Step 3a>
the step of bringing at least a part of the medical device substrate into contact with a solution of an ionic polymer and then removing a surplus polymer solution.

14. A method of manufacturing a medical device according to any one of claims 1 to 12, the method comprising the following Steps 1a to 3a in this order:

<Step 1a>
the step of synthesizing an ionic block polymer having a hydrophobic segment;
<Step 2a>
the step of bringing at least a part of a medical device substrate into contact with a solution of an ionic block polymer having a hydrophobic segment and then removing a surplus polymer solution;
<Step 3a>
the step of bringing at least a part of the medical device substrate into contact with a solution of an ionic polymer and then removing a surplus polymer solution.

15. The method according to claim 13 or 14, wherein the ionic polymer in the Step 3a has a charge opposite to the ionic block polymer having a hydrophobic segment.

[Fig 1]

[Fig 1]

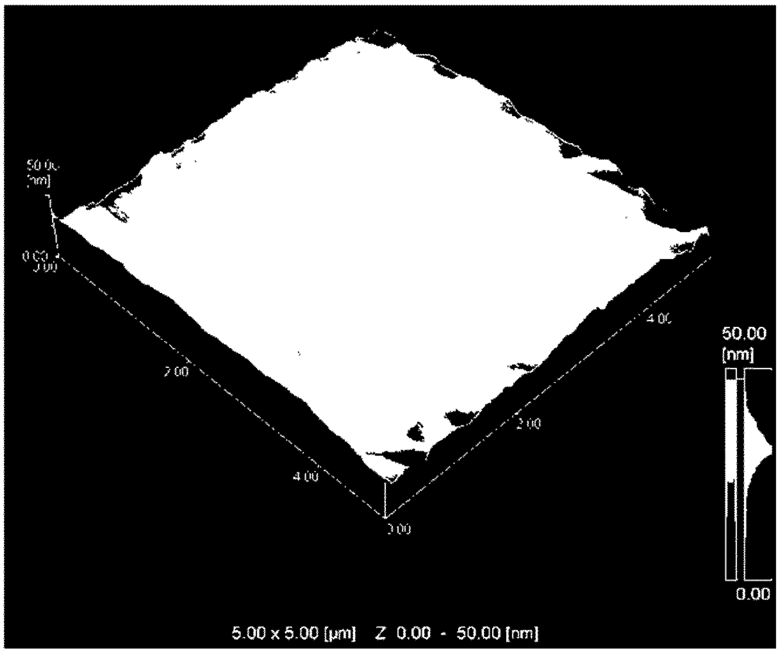


[Fig 2]

[Fig 2]

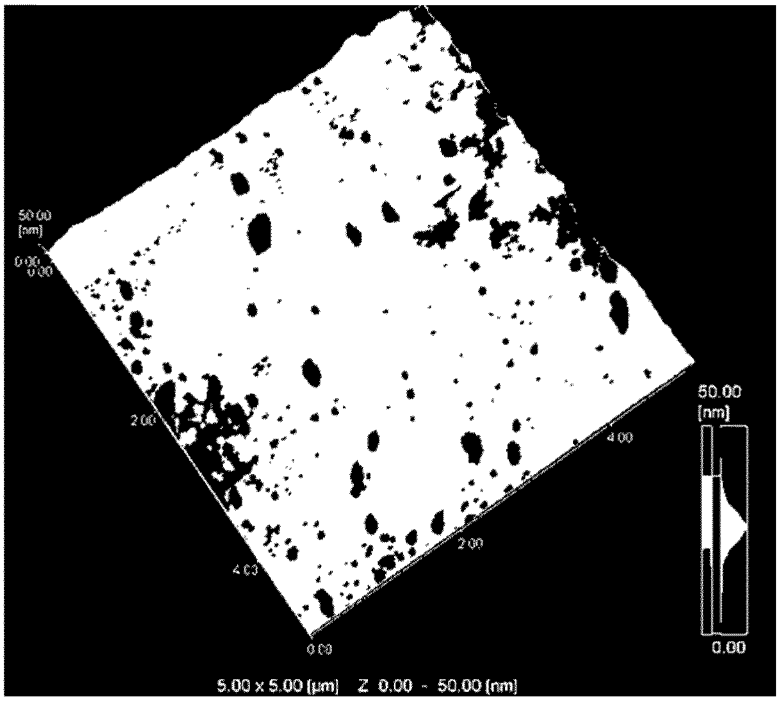

**INTERNATIONAL SEARCH REPORT**

International application No. PCT/JP2017/027184

A. CLASSIFICATION OF SUBJECT MATTER
*A61L27/14*(2006.01)i, *A61L27/44*(2006.01)i, *A61L27/50*(2006.01)i, *G02C7/04*(2006.01)i

According to International Patent Classification (IPC) or to both national classification and IPC

B. FIELDS SEARCHED

Minimum documentation searched (classification system followed by classification symbols)
A61L27/14, A61L27/44, A61L27/50, G02C7/04

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2017 |
| Kokai Jitsuyo Shinan Koho | 1971-2017 | Toroku Jitsuyo Shinan Koho | 1994-2017 |

Electronic data base consulted during the international search (name of data base and, where practicable, search terms used)
JSTPlus/JMEDPlus/JST7580(JDreamIII), CAplus/REGISTRY/MEDLINE/EMBASE/BIOSIS(STN)

C. DOCUMENTS CONSIDERED TO BE RELEVANT

| Category* | Citation of document, with indication, where appropriate, of the relevant passages | Relevant to claim No. |
|---|---|---|
| X/Y | JP 2012-501972 A (Abbott Cardiovascular Systems, Inc.), 26 January 2012 (26.01.2012), claim 1; paragraphs [0057], [0059] & WO 2010/028087 A2 claim 1; page 15, 1st paragraph; page 16, 2nd paragraph & US 2010/0057037 A1 & EP 2331154 A1 | 1-6,8,13-15/ 7-10 |

☒ Further documents are listed in the continuation of Box C.  ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 28 September 2017 (28.09.17) | 10 October 2017 (10.10.17) |

| Name and mailing address of the ISA/ | Authorized officer |
|---|---|
| Japan Patent Office<br>3-4-3,Kasumigaseki,Chiyoda-ku,<br>Tokyo 100-8915,Japan | Telephone No. |

Form PCT/ISA/210 (second sheet) (January 2015)

**INTERNATIONAL SEARCH REPORT**

International application No.
PCT/JP2017/027184

C (Continuation). DOCUMENTS CONSIDERED TO BE RELEVANT

| Category* | Citation of document, with indication, where appropriate, of the relevant passages | Relevant to claim No. |
|---|---|---|
| Y | JP 2014-514422 A (Johnson & Johnson Vision Care, Inc.), 19 June 2014 (19.06.2014), claims 6, 22, 23; examples 3, 11, 14 & WO 2012/151134 A1 claims 6, 22, 23; examples 3, 11, 14 & US 2012/0283381 A1 & EP 2705073 A1 & CN 103502317 A & KR 10-2014-0025504 A | 6-15 |
| X/Y | JP 2004-535227 A (Novartis AG.), 25 November 2004 (25.11.2004), claims; paragraphs [0074], [0035], [0037] & WO 2002/096477 A2 claims; example 1 & US 2003/0039742 A1 & EP 1429818 A2 | 1-5,8,11-15/ 6-15 |
| X/Y | JP 2005-538418 A (Novartis AG.), 15 December 2005 (15.12.2005), claims; paragraphs [0001], [0002], [0030], [0047] & WO 2004/025332 A1 claims; page 1, 1st to 2nd paragraphs; page 7, 3rd paragraph; page 8, the last paragraph & US 2004/0047979 A1 & EP 1540381 A1 | 1-5,8-15/ 6-15 |
| P,X | WO 2016/148146 A1 (Toray Industries, Inc.), 22 September 2016 (22.09.2016), a whole article (Family: none) | 1-15 |
| A | TABUCHI N., et al, Adsorption of actives in ophthalmological drugs for over-the-counter on soft contact lens surfaces. J Oleo Sci., 2009, Vol.58 No.1, p.43-52 (ISSN 1345-8957) (abstract) | 1-15 |

Form PCT/ISA/210 (continuation of second sheet) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2013024801 A
- JP 2005538767 A
- JP 2013532196 A
- JP 2012246489 A
- JP 2007206166 A
- WO 9935520 A
- WO 0157118 A
- US 20010045676 A1
- JP 2014514422 A
- WO 2011102356 A